# EUROPÄISCHE PATENTSCHRIFT

(11) **EP 3 427 707 B1**
(45) Veröffentlichungstag und Bekanntmachung des Hinweises auf die Patenterteilung: **22.04.2020**
(21) Anmeldenummer: 17020302.0
(22) Anmeldetag: 13.07.2017
(51) Int. Cl.: A61F 9/008

(54) **VORRICHTUNG ZUM BEARBEITEN VON AUGENGEWEBE MITTELS EINES GEPULSTEN LASERSTRAHLS**
DEVICE FOR TREATING EYE TISSUE USING A PULSED LASER BEAM
DISPOSITIF DE TRAITEMENT DE TISSU OCULAIRE À L'AIDE D'UN RAYON LASER PULSÉ

(43) Veröffentlichungstag der Anmeldung: 16.01.2019
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- DE-A1-102007 019 813
- DE-A1-102011 108 645
- DE-A1-102012 022 081
- US-A1- 2014 128 856

## Beschreibung

### Technisches Gebiet

Die vorliegende Erfindung betrifft eine ophthalmologische Vorrichtung zum Bearbeiten von Augengewebe mittels eines gepulsten Laserstrahls. Die vorliegende Erfindung betrifft insbesondere eine ophthalmologische Vorrichtung mit einer Laserquelle zur Erzeugung eines gepulsten Laserstrahls, einer Fokussieroptik zur Fokussierung des gepulsten Laserstrahls ins Augengewebe und ein Scannersystem, um den gepulsten Laserstrahl auf einen Bearbeitungszielpunkt im Augengewebe zu lenken.

### Stand der Technik

Zum Bearbeiten von Augengewebe mittels eines Laserstrahls wird ein Bearbeitungsbereich mit Laserpulsen gescannt (abgetastet), indem der gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern, Polygonscannern oder Resonanzscannern.

In US 7,621,637 wird eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels einer mechanisch bewegten Projektionsoptik einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung der Projektionsoptik und somit auf dessen Scannmuster überlagert. Ein Fast-Scannersystem in der Basisstation ermöglicht eine feine Bewegung der Laserpulse (Microscann), welche auf das Scannmuster der beweglichen Projektionsoptik überlagert wird, die einen grossen Bearbeitungsbereich abdeckt, beispielsweise das gesamte Auge.

Derartige bekannte Systeme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei Anwendungen, in denen nicht nur Gewebeschnitte in einer im Wesentlichen horizontal ausgerichteten Bearbeitungsfläche auf einer gemeinsamen Fokalfläche ausgeführt werden, sondern in denen auch Schnitte mit einer vertikalen Schnittkomponente über unterschiedliche Fokushöhen ausgeführt werden sollen, z.B. schräg zur Horizontalen verlaufende oder vertikale Schnitte, erweist sich das vertikale Verfahren der Projektionsoptik oder eines Zoom-Systems für eine vertikale Veränderung des Fokus und damit der Schnitthöhe als zu langsam um Schnitte mit einer vertikalen Komponente, also mit veränderlicher Fokustiefe während des Schneidens, mit einer Geschwindigkeit auszuführen, die vergleichbar ist mit Schnittgeschwindigkeiten in der horizontalen Bearbeitungsfläche.

US 2016/0089270 beschreibt ein System und Verfahren zum Schneiden von Lentikeln im Augengewebe. Gemäss US 2016/0089270 werden dazu gradlinige Fastscannlinien langsameren Bearbeitungslinien überlagert, die entlang Meridianen des Lentikels abgefahren werden. Durch die Gradlinigkeit der Fastscannlinien entstehen Schnitte, die in ihrer Form von der gewünschten Oberflächenkrümmung des Lentikels abweichen und daher Fehler verursachen. Überdies ist zum Abfahren der Bearbeitungslinien entlang der Meridiane jeweils über die Distanz einer Lentikelbreite eine vertikale Fokusverschiebung in der Grössenordnung und im Ausmass der Dicke des zu schneidenden Lentikels erforderlich, was einerseits mit entsprechendem Aufwand und Kosten für dazu eingerichtete verschiebbare Optiken und bewegliche Linsen und andererseits mit damit einhergehenden Einbussen in der Bearbeitungsgeschwindigkeit verbunden ist. Darüber hinaus erlauben die Fastscannlinien aufgrund ihrer festen horizontalen Ausrichtung keine bestmögliche Anpassung von Schnitten an Lentikeloberflächen, insbesondere nicht, wenn diese von einer
sphärischen Form abweichen.

In DE102012022081 wird ein Lentikel mittels Laser geschnitten, welches einen vorhergehenden Cap-Schnitt einer Behandlung berücksichtigt und als obere Schnittfläche für den neuen Lentikelschnitt benutzt. Dafür wird dieser alte Cap-Schnitt vermessen, da er sich mit der Zeit verändert haben kann. Die tatsächliche Lage wird berücksichtigt in der neuen Berechnung der unteren Lentikelfläche. In DE102012022081 werden auch mögliche Verformungen an dem ursprünglichen Schnitt berücksichtigt, jedoch sind dieses Langzeit-Verformungen durch Epithelisierung, Vernarbung usw. Kurzzeitige Verformungen während des Schneidprozesses werden nicht berücksichtigt.

### Darstellung der Erfindung

Es ist eine Aufgabe der vorliegenden Erfindung eine Vorrichtung zum Bearbeiten von Augengewebe mittels eines gepulsten Laserstrahls vorzuschlagen, welche zumindest einige Nachteile des Stands der Technik nicht aufweist.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

In einem ersten Aspekt der vorliegenden Erfindung umfasst eine ophthalmologische Vorrichtung zum Bearbeiten von Augengewebe eine Laserquelle, die eingerichtet ist, einen gepulsten Laserstrahl zu erzeugen; eine Fokussieroptik, die eigerichtet ist, den gepulsten Laserstrahl ins Augengewebe zu fokussieren; und ein Scannersystem, das eingerichtet ist, den gepulsten Laserstrahl auf Bearbeitungszielpunkte im Augengewebe zu lenken; ein Messsystem, das eingerichtet ist, Strukturen im Augengewebe optisch zu erfassen; und einen Schaltkreis, der eingerichtet ist, das Scannersystem derart zu steuern, dass das Scannersystem den gepulsten Laserstrahl auf Bearbeitungszielpunkte auf einer ersten Aussenfläche eines im Augengewebe zu schneidenden Lentikels lenkt, um einen ersten Gewebeschnitt zum Schneiden des Lentikels zu erzeugen; und die oben genannten Ziele werden wenigstens teilweise dadurch erreicht, dass der Schaltkreis überdies eingerichtet ist, das Messsystem derart zu steuern, dass das Messsystem die durch den ersten Gewebeschnitt erzeugte erste Aussenfläche des Lentikels erfasst, und das Scannersystem derart zu steuern, dass das Scannersystem den gepulsten Laserstrahl auf Bearbeitungszielpunkte auf einer bezüglich der erfassten ersten Aussenfläche des Lentikels positionierten zweiten Aussenfläche des zu schneidenden Lentikels lenkt, um einen bezüglich der erfassten ersten Aussenfläche des Lentikels positionierten zweiten Gewebeschnitt zum Schneiden des Lentikels zu erzeugen. In einer Ausführungsvariante ist das Messsystem als interferometrisches Messsystem ausgeführt. Die Erfassung eines Gewebeschnitts auf einer ersten Aussenfläche eines zu schneidenden Lentikels und die davon abhängige Positionierung und Ausführung eines Gewebeschnitts auf einer zweiten Aussenfläche des Lentikels ermöglicht eine flexible und präzise Anpassung von Form und Lage des durchzuführenden Gewebeschnitts respektive der zweiten Aussenfläche an die tatsächliche Lage und Form des bereits durchgeführten Gewebeschnitts und bewirkt dadurch eine Verbesserung von Form und Grösse des Lentikels, insbesondere von dessen Dicke, und der damit erreichbaren erwünschten refraktiven Korrektur des Auges. In einer Ausführungsvariante ist der Schaltkreis eingerichtet, die zweite Aussenfläche des zu schneidenden Lentikels mit einer vorgegebenen Zentrumsdicke des zu schneidenden Lentikels bezüglich der erfassten ersten Aussenfläche zu positionieren.

In einer weiteren Ausführungsvariante ist der Schaltkreis eingerichtet, die zweite Aussenfläche des zu schneidenden Lentikels mit einem vorgegebenen Dickenprofil des zu schneidenden Lentikels bezüglich der erfassten ersten Aussenfläche zu positionieren.

In einer Ausführungsvariante ist der Schaltkreis eingerichtet, das Messsystem derart zu steuern, dass das Messsystem durch beim ersten Gewebeschnitt erzeugte Gasblasen bewirkte Verformungen der ersten Aussenfläche erfasst, und die zweite Aussenfläche des zu schneidenden Lentikels unter Berücksichtigung der erfassten Verformungen bezüglich der erfassten ersten Aussenfläche positioniert.

In einer weiteren Ausführungsvariante ist der Schaltkreis eingerichtet, die erste Aussenfläche des zu schneidenden Lentikels als eine von einer äusseren Hornhautoberfläche abgewandte Unterseite des zu schneidenden Lentikels zu bestimmen, und die zweite Aussenfläche des zu schneidenden Lentikels als eine von der äusseren Hornhautoberfläche zugewandte Oberseite des zu schneidenden Lentikels zu bestimmen.

In einer Ausführungsvariante ist der Schaltkreis eingerichtet, das Scannersystem derart zu steuern, dass das Scannersystem den gepulsten Laserstrahl auf Bearbeitungszielpunkte in nebeneinander verlaufenden Bearbeitungsbahnen auf der ersten Aussenfläche des zu schneidenden Lentikels lenkt, um nebeneinander verlaufende Schnittbahnen des ersten Gewebeschnitts zu erzeugen; und das Messsystem derart zu steuern, dass das Messsystem die nebeneinander verlaufenden Schnittbahnen erfasst und die zweite Aussenfläche des zu schneidenden Lentikels bezüglich der erfassten nebeneinander verlaufenden Schnittbahnen positioniert.

In einer weiteren Ausführungsvariante ist der Schaltkreis eingerichtet, das Scannersystem derart zu steuern, dass das Scannersystem den gepulsten Laserstrahl auf Bearbeitungszielpunkte in nebeneinander verlaufenden Bearbeitungsbahnen auf der ersten Aussenfläche des zu schneidenden Lentikels lenkt, um nebeneinander verlaufende, durch verbleibende Gewebebrücken getrennte Schnittbahnen des ersten Gewebeschnitts zu erzeugen; das Messsystem derart zu steuern, dass das Messsystem die Schnittbahnen erfasst; und das Scannersystem derart zu steuern, dass das Scannersystem den gepulsten Laserstrahl auf Bearbeitungszielpunkte in die basierend auf den erfassten Schnittbahnen bestimmten verbleibenden Gewebebrücken auf der ersten Aussenfläche lenkt, um den ersten Gewebeschnitt zu erzeugen.

In einer Ausführungsvariante ist der Schaltkreis eingerichtet, das Scannersystem derart zu steuern, dass das Scannersystem den gepulsten Laserstrahl auf Bearbeitungszielpunkte in Bearbeitungsbahnen lenkt, die auf der Aussenfläche des Lentikels verlaufen und eine gradlinige, entlang von Kreisen verlaufende, konzentrisch kreisförmige, konzentrisch elliptische, spiralförmige oder spiralarmförmige Ausgestaltung aufweist.

In einer weiteren Ausführungsvariante ist der Schaltkreis eingerichtet, ein zweites Scannermodul des Scannersystems derart zu steuern, dass das zweite Scannermodul den gepulsten Laserstrahl auf Bearbeitungszielpunkte in einer Vorschubrichtung entlang einer Bearbeitungslinie lenkt, die auf der ersten respektive zweiten Aussenfläche des zu schneidenden Lentikels verläuft; dass das Scannersystem ein erstes Scannermodul umfasst, das eingerichtet ist, den gepulsten Laserstrahl entlang einer um einen Ausrichtungswinkel quer zur Bearbeitungslinie in einer horizontalen Bearbeitungsebene verlaufenden Scannlinie zu lenken, mit einer wesentlich höheren Scanngeschwindigkeit im Vergleich zur Scanngeschwindigkeit des zweiten Scannermoduls in Vorschubrichtung; und dass das Scannersystem einen z-Modulator umfasst, der eingerichtet ist, die Scannlinie abhängig von einem bestimmten Bearbeitungszielpunkt des zweiten Scannermoduls auf der Bearbeitungslinie derart aus der Bearbeitungsebene zu verkippen, dass die Scannlinie im Wesentlichen entlang der Aussenfläche des Lentikels verläuft.

In einem zweiten Aspekt der vorliegenden Erfindung umfasst die ophthalmologische Vorrichtung zum Bearbeiten von Augengewebe eine Laserquelle, die eingerichtet ist, einen gepulsten Laserstrahl zu erzeugen; eine Fokussieroptik, die eigerichtet ist, den gepulsten Laserstrahl ins Augengewebe zu fokussieren; und ein Scannersystem, das eingerichtet ist, den gepulsten Laserstrahl auf Bearbeitungszielpunkte im Augengewebe zu lenken; und die oben genannten Ziele werden wenigstens teilweise dadurch erreicht, dass der Schaltkreis eingerichtet, das Scannersystem derart zu steuern, dass das Scannersystem den gepulsten Laserstrahl auf Bearbeitungszielpunkte lenkt auf eine in einer Hornhaut zu schneidende Fläche, in nebeneinander verlaufenden Bearbeitungsbahnen, um zunächst durch verbleibende Gewebebrücken getrennte Schnittbahnen eines auf der Fläche vorzunehmenden Gewebeschnitts zu erzeugen, und danach den gepulsten Laserstrahl auf Bearbeitungszielpunkte in die verbleibenden Gewebebrücken zwischen den Schnittbahnen auf der Fläche lenkt, um den Gewebeschnitt zu vervollständigen. Das Schneiden von durch Gewebebrücken getrennten Schnittbahnen ermöglicht jeweils eine neue Schnittbahn zu schneiden, ohne dass diese durch Verformungen beeinträchtigt wird, die durch Gasbildung in einer bereits geschnittenen direkt benachbarten Schnittbahn verursacht werden.

In einer Ausführungsvariante umfasst die ophthalmologische Vorrichtung ein Messsystem, das eingerichtet ist, Strukturen im Augengewebe optisch zu erfassen. Der Schaltkreis ist eingerichtet, das Messsystem derart zu steuern, dass das Messsystem die erzeugten Schnittbahnen erfasst, und die Bearbeitungszielpunkte in den verbleibenden Gewebebrücken basierend auf den erfassten Schnittbahnen positioniert. In einer Ausführungsvariante ist das Messsystem als interferometrisches Messsystem ausgeführt. Die Erfassung bereits geschnittener Schnittbahnen und die Bearbeitung von verbliebenen Gewebebrücken unter Berücksichtigung der bereits geschnittenen Schnittbahnen ermöglicht eine flexible Anpassung an die tatsächliche Form durchgeführter Schnittbahnen und dadurch eine Vermeidung oder zumindest Reduktion von überlappenden Schnittbahnen über ausgedehnte Gebiete.

In einer weiteren Ausführungsvariante ist der Schaltkreis eingerichtet, das Scannersystem derart zu steuern, dass das Scannersystem den gepulsten Laserstrahl mit sich überlappenden Laserpulsspots auf aufeinanderfolgende Bearbeitungszielpunkte lenkt.

In einer Ausführungsvariante ist der Schaltkreis eingerichtet, das Scannersystem derart zu steuern, dass das Scannersystem den gepulsten Laserstrahl auf Bearbeitungszielpunkte in den nebeneinander verlaufenden Bearbeitungsbahnen in die verbleibenden Gewebebrücken lenkt.

In einer weiteren Ausführungsvariante ist der Schaltkreis eingerichtet, das Scannersystem derart zu steuern, dass das Scannersystem den gepulsten Laserstrahl in den verbleibenden Gewebebrücken auf Bearbeitungszielpunkte in Bearbeitungsbahnen lenkt, welche eine über die Gewebebrücken hinausgehende Breite aufweisen.

In einer Ausführungsvariante ist der Schaltkreis eingerichtet, das Scannersystem derart zu steuern, dass das Scannersystem den gepulsten Laserstrahl auf Bearbeitungszielpunkte in parallel nebeneinander verlaufenden Bearbeitungsbahnen lenkt.

In einer weiteren Ausführungsvariante ist der Schaltkreis eingerichtet, das Scannersystem derart zu steuern, dass das Scannersystem den gepulsten Laserstrahl auf Bearbeitungszielpunkte in nebeneinander verlaufenden Bearbeitungsbahnen lenkt, die eine spiralförmige, kreisförmige oder elliptische Form aufweisen.

In einer Ausführungsvariante ist der Schaltkreis eingerichtet, das Scannersystem derart zu steuern, dass das Scannersystem den gepulsten Laserstrahl in einer Vorschubrichtung auf Bearbeitungszielpunkte entlang einer in den Bearbeitungsbahnen verlaufenden Bearbeitungslinie lenkt. Das Scannersystem ist eingerichtet, den gepulsten Laserstrahl entlang einer quer zur Bearbeitungslinie verlaufenden Scannlinie innerhalb der Bearbeitungsbahnen zu lenken, mit einer wesentlich höheren Scanngeschwindigkeit im Vergleich zur Scanngeschwindigkeit in Vorschubrichtung.

In einer weiteren Ausführungsvariante ist der Schaltkreis eingerichtet, das Scannersystem derart zu steuern, dass das Scannersystem den gepulsten Laserstrahl auf Bearbeitungszielpunkte auf einer Aussenfläche eines in einer Hornhaut zu schneidenden Lentikels lenkt, in den nebeneinander verlaufenden Bearbeitungsbahnen, um die durch verbleibende Gewebebrücken getrennten Schnittbahnen des vorzunehmenden Gewebeschnitts an der Aussenfläche des Lentikels zu erzeugen, und danach den gepulsten Laserstrahl auf Bearbeitungszielpunkte in die verbleibenden Gewebebrücken zwischen den Schnittbahnen auf der Aussenfläche des Lentikels lenkt, um den Gewebeschnitt zu vervollständigen.

In einer Ausführungsvariante ist der Schaltkreis eingerichtet, das Scannersystem derart zu steuern, dass das Scannersystem den gepulsten Laserstrahl auf Bearbeitungszielpunkte auf die von einer äusseren Hornhautoberfläche abgewandte untere Aussenfläche des zu schneidenden Lentikels lenkt, um die durch verbleibende Gewebebrücken getrennten Schnittbahnen auf der unteren Aussenfläche des zu schneidenden Lentikels zu erzeugen, und dass das Scannersystem den gepulsten Laserstrahl danach auf Bearbeitungszielpunkte lenkt auf einer von der äusseren Hornhautoberfläche zugewandten oberen Aussenfläche des zu schneidenden Lentikels, in nebeneinander verlaufenden Bearbeitungsbahnen, um durch verbleibende Gewebebrücken getrennte Schnittbahnen eines an der oberen Aussenfläche des Lentikels vorzunehmenden oberen Gewebeschnitts zu erzeugen, bevor das Scannersystem den gepulsten Laserstrahl auf die Bearbeitungszielpunkte in die verbleibenden Gewebebrücken auf der unteren Aussenfläche des Lentikels lenkt, um den unteren Gewebeschnitt zu vervollständigen; und dass das Scannersystem danach den gepulsten Laserstrahl auf Bearbeitungszielpunkte in die verbleibenden Gewebebrücken auf der oberen Aussenfläche des Lentikels lenkt, um den oberen Gewebeschnitts zu vervollständigen.

In einer weiteren Ausführungsvariante umfasst die ophthalmologische Vorrichtung ein Messsystem, das eingerichtet ist, Strukturen im Augengewebe optisch zu erfassen. Der Schaltkreis ist eingerichtet, das Messsystem derart zu steuern, dass das Messsystem die erzeugten Schnittbahnen des vorzunehmenden unteren Gewebeschnitts erfasst, und die Bearbeitungsbahnen des vorzunehmenden oberen Gewebeschnitts bezüglich der erfassten Schnittbahnen des unteren Gewebeschnitts positioniert. In einer Ausführungsvariante ist das Messsystem als interferometrisches Messsystem ausgeführt.

In einer weiteren Ausführungsvariante ist der Schaltkreis eingerichtet, die Bearbeitungsbahnen des vorzunehmenden oberen Gewebeschnitts bezüglich der erfassten Schnittbahnen des unteren Gewebeschnitts mit kleinerer Bahnbreite zu bestimmen.

In einer Ausführungsvariante ist der Schaltkreis eingerichtet, die Bearbeitungsbahnen zur Vervollständigung des unteren Gewebeschnitts bezüglich der erfassten Schnittbahnen des unteren Gewebeschnitts zu positionieren.

In einem dritten Aspekt der vorliegenden Erfindung umfasst die ophthalmologische Vorrichtung zum Bearbeiten von Augengewebe, insbesondere zum Schneiden eines Lentikels im Augengewebe, eine Laserquelle, die eingerichtet ist, einen gepulsten Laserstrahl zu erzeugen; eine Fokussieroptik, die eigerichtet ist, den gepulsten Laserstrahl ins Augengewebe zu fokussieren; ein Scannersystem, umfassend ein erstes Scannermodul und ein zweites Scannermodul, zum Ablenken des gepulsten Laserstrahls auf Bearbeitungszielpunkte im Augengewebe; und einen Schaltkreis zur Steuerung der ophthalmologischen Vorrichtung; und die oben genannten Ziele werden wenigstens teilweise dadurch erreicht, dass der Schaltkreis eingerichtet ist, das zweite Scannermodul derart zu steuern, dass das zweite Scannermodul den gepulsten Laserstrahl in einer Vorschubrichtung auf Bearbeitungszielpunkte entlang einer Bearbeitungslinie lenkt, die auf einer Aussenfläche eines im Augengewebe zu schneidenden Lentikels quer zu Meridianen des Lentikels verläuft. Das erste Scannermodul ist eingerichtet, den gepulsten Laserstrahl auf Bearbeitungszielpunkte entlang einer um einen Ausrichtungswinkel quer zur Bearbeitungslinie in einer horizontalen Bearbeitungsebene verlaufenden Scannlinie zu lenken, mit einer wesentlich höheren Scanngeschwindigkeit im Vergleich zur Scanngeschwindigkeit des zweiten Scannermoduls in Vorschubrichtung. Die ophthalmologische Vorrichtung umfasst einen z-Modulator, der eingerichtet ist, die Scannlinie abhängig von einem bestimmten Bearbeitungszielpunkt des zweiten Scannermoduls auf der Bearbeitungslinie derart aus der Bearbeitungsebene zu verkippen, dass die Scannlinie im Wesentlichen entlang der Aussenfläche des Lentikels verläuft. Durch diese vom aktuellen Bearbeitungszielpunkt der (vergleichsweise langsamer gescannten) Bearbeitungslinie abhängige Verkippung der (vergleichsweise schneller gescannten) Sannlinie können im Augengewebe Lentikel mit mehreren nebeneinanderliegenden aus der horizontalen Bearbeitungsebene verkippten Schnittbahnen geschnitten werden, ohne dass dabei wesentliche Abweichungen zur Oberflächenkrümmung des zu schneidenden Lentikels erzeugt werden. Insbesondere im Vergleich zu Lösungen mit fest horizontal ausgerichteten Scannlinien, die keine optimale Schnittanpassung an Oberflächenkrümmungen von Lentikeln erlauben, ermöglicht die dynamische Änderung der Scannlinienverkippung eine flexiblere und präzisere Schnittanpassung an Lentikeloberflächen mit örtlich variierender Oberflächenkrümmung. Abhängig von der gewählten Form der Bearbeitungslinie, können die Lentikel im Augengewebe überdies geschnitten werden, ohne dass dazu vertikale Fokusverschiebungen in der Grössenordnung und im Ausmass der gesamten Tiefe der durchzuführenden vertikalen Schnittkomponente, also der Dicke des zu schneidenden Lentikels, durch Verschiebungen der Projektionsoptik oder Bewegungen von Linsen eines Zoomsystems mit einer Geschwindigkeit vorgenommen werden müssen, mit der das zweite Scannermodul eine der lateralen Ausdehnung des zu schneidenden Lentikels entsprechende Distanz der Bearbeitungslinie abfährt.

In einer Ausführungsvariante umfasst die ophthalmologische Vorrichtung einen Rotator, der eingerichtet ist, eine durch die Scannlinie und den gepulsten Laserstrahl definierte Fastscannebene derart um eine optische Übertragungsachse zu drehen, dass der Ausrichtungswinkel der Scannlinie zur Bearbeitungslinie verändert wird.

In einer weiteren Ausführungsvariante ist der Schaltkreis eingerichtet, den Rotator abhängig vom bestimmten Bearbeitungszielpunkt des zweiten Scannermoduls auf der Bearbeitungslinie zu steuern, so dass der Ausrichtungswinkel der Scannlinie zur Bearbeitungslinie abhängig vom bestimmten Bearbeitungszielpunkt des zweiten Scannermoduls auf der Bearbeitungslinie eingestellt wird.

In einer Ausführungsvariante umfasst die ophthalmologische Vorrichtung einen Scannlängenmodulator, der eingerichtet ist, eine Länge der Scannlinie zu verändern. Der Schaltkreis ist eingerichtet, den Scannlängenmodulator abhängig vom bestimmten Bearbeitungszielpunkt des zweiten Scannermoduls auf der Bearbeitungslinie derart zu steuern, dass die Länge der Scannlinie abhängig vom bestimmten Bearbeitungszielpunkt des zweiten Scannermoduls auf der Bearbeitungslinie eingestellt wird.

In einer weiteren Ausführungsvariante ist der Schaltkreis eingerichtet, das zweite Scannermodul derart zu steuern, dass das zweite Scannermodul den gepulsten Laserstrahl auf Bearbeitungszielpunkte in der Bearbeitungsebene entlang einer kreisförmigen oder elliptischen Bearbeitungslinie lenkt, die auf der Aussenfläche des Lentikels verläuft.

In einer Ausführungsvariante ist der Schaltkreis eingerichtet, das zweite Scannermodul derart zu steuern, dass das zweite Scannermodul den gepulsten Laserstrahl nacheinander entlang mehrerer in übereinanderliegenden Bearbeitungsebenen angeordneten kreisförmigen oder elliptischen Bearbeitungslinien lenkt, die auf der Aussenfläche des Lentikels verlaufen.

In einer weiteren Ausführungsvariante ist der Schaltkreis eingerichtet, das zweite Scannermodul derart zu steuern, dass das zweite Scannermodul den gepulsten Laserstrahl auf Bearbeitungszielpunkte entlang einer spiralförmigen Bearbeitungslinie lenkt, die auf der Aussenfläche des Lentikels verläuft.

In einer Ausführungsvariante ist der Schaltkreis eingerichtet, das zweite Scannermodul derart zu steuern, dass das zweite Scannermodul den gepulsten Laserstrahl auf Bearbeitungszielpunkte entlang einer Bearbeitungslinie lenkt, die in der der Form eines Kreisbogensegments, eines Spiralbogensegments oder eines gekrümmten Liniensegments auf der Aussenfläche des Lentikels verläuft.

In einer weiteren Ausführungsvariante ist der Schaltkreis eingerichtet, das zweite Scannermodul derart zu steuern, dass das zweite Scannermodul den gepulsten Laserstrahl auf Bearbeitungszielpunkte entlang mehrerer spiralförmiger Bearbeitungslinien lenkt, die in einem ersten Schritt ausgehend von einer Peripherie der Aussenfläche des Lentikels spiralarmförmig in Richtung eines Zentrums der Aussenfläche verlaufen, und in einem zweiten Schritt ausgehend vom Zentrum spiralarmförmig zur Peripherie der Aussenfläche des Lentikels verlaufen.

In einer Ausführungsvariante ist der Schaltkreis eingerichtet, das zweite Scannermodul derart zu steuern, dass das zweite Scannermodul den gepulsten Laserstrahl auf Bearbeitungszielpunkte entlang mehrerer spiralförmiger Bearbeitungslinien lenkt, die in einem ersten Schritt ausgehend von einer Peripherie der Aussenfläche des Lentikels spiralarmförmig in Richtung eines Zentrums der Aussenfläche verlaufen und mit einem bestimmten Abstand zum Zentrum enden, und in einem zweiten Schritt ausgehend vom bestimmten Abstand zum Zentrum spiralarmförmig zur Peripherie der Aussenfläche des Lentikels verlaufen, oder umgekehrt im ersten Schritt ausgehend vom bestimmten Abstand zum Zentrum spiralarmförmig zur Peripherie der Aussenfläche des Lentikels verlaufen und von der Peripherie der Aussenfläche des Lentikels spiralarmförmig in Richtung des Zentrums der Aussenfläche verlaufen.

In einer weiteren Ausführungsvariante ist der z-Modulator eingerichtet, die Scannlinie bezüglich der Bearbeitungsebene zu krümmen. Der Schaltkreis ist eingerichtet, den z-Modulator abhängig vom bestimmten Bearbeitungszielpunkt des zweiten Scannermoduls auf der Bearbeitungslinie derart zu steuern, dass der z-Modulator die Scannlinie abhängig vom bestimmten Bearbeitungszielpunkt des zweiten Scannermoduls auf der Bearbeitungslinie zur Anpassung an die Aussenfläche des Lentikels krümmt.

In einer Ausführungsvariante umfasst die ophthalmologische Vorrichtung einen Rotator, der eingerichtet ist, eine durch die Scannlinie und den gepulsten Laserstrahl definierte Fastscannebene um eine optische Übertragungsachse zu drehen, um den Ausrichtungswinkel der Scannlinie zur Bearbeitungslinie zu verändern. Der z-Modulator ist eingerichtet, die Scannlinie bezüglich der Bearbeitungsebene zu krümmen. Die ophthalmologische Vorrichtung umfasst einen Scannlängenmodulator, der eingerichtet ist, eine Länge der Scannlinie zu verändern. Der Schaltkreis ist eingerichtet, abhängig vom bestimmten Bearbeitungszielpunkt des zweiten Scannermoduls auf der Bearbeitungslinie den Rotator zum Einstellen des Ausrichtungswinkels der Scannlinie zur Bearbeitungslinie, den z-Modulator zur Krümmung der Scannlinie bezüglich der Bearbeitungsebene, und den Scannlängenmodulator zum Einstellen der Länge der Scannlinie derart zu steuern, dass die Aussenfläche des Lentikels in vorgegebener Freiform zur Korrektur einer Aberration höherer Ordnung geschnitten wird.

In einer weiteren Ausführungsvariante umfasst die ophthalmologische Vorrichtung einen Rotator, der eingerichtet ist, eine durch die Scannlinie und den gepulsten Laserstrahl definierte Fastscannebene um eine optische Übertragungsachse zu drehen, um den Ausrichtungswinkel der Scannlinie zur Bearbeitungslinie zu verändern. Die ophthalmologische Vorrichtung umfasst einen Scannlängenmodulator, der eingerichtet ist, eine Länge der Scannlinie zu verändern. Der Schaltkreis ist eingerichtet, abhängig vom bestimmten Bearbeitungszielpunkt des zweiten Scannermoduls auf der Bearbeitungslinie den Rotator zum Einstellen des Ausrichtungswinkels der Scannlinie zur Bearbeitungslinie, den z-Modulator zur Verkippung der Scannlinie bezüglich der Bearbeitungsebene, und den Scannlängenmodulator zum Einstellen der Länge der Scannlinie derart zu steuern, dass die Aussenfläche des Lentikels in vorgegebener Freiform zur Korrektur einer Aberration höherer Ordnung geschnitten wird.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagramm, das schematisch eine ophthalmologische Vorrichtung zur Bearbeitung von Augengewebe mit einem gepulsten Laserstrahl illustriert, welche ein Scannersystem zum Abtasten des Augengewebes mit dem gepulsten Laserstrahl an Bearbeitungszielpunkten im Augengewebe umfasst.
- Figur 2:: zeigt ein Blockdiagramm der ophthalmologischen Vorrichtung, welches mehrere Module des Scannersystems illustriert, die dazu beitragen, den gepulsten Laserstrahl entlang einer quer zu einer Bearbeitungslinie verlaufenden Scannlinie zu lenken.
- Figur 2a:: zeigt eine schematische Aufsicht eines Augengewebebereichs, welche eine Bearbeitungslinie in Vorschubrichtung und eine quer zur Bearbeitungslinie verlaufende Scannlinie illustriert.
- Figur 2b:: zeigt eine schematische Querschnittsansicht eines Augengewebebereichs, welche eine Bearbeitungslinie illustriert, die in einer senkrecht zur Zeichenebene verlaufenden Bearbeitungsebene verläuft, und eine aus der Bearbeitungsebene verkippte Scannlinie.
- Figur 3:: zeigt einen schematischen Querschnitt eines Abschnitts des Strahlengangs in einem Divergenzmodulator mit mindestens einer verschiebbaren Linse und illustriert die durch die Verschiebung der Linse veränderte Divergenz des Laserstrahls.
- Figur 4:: zeigt eine schematische Querschnittsansicht eines Augengewebebereichs mit einem in der Cornea geschnittenen Lentikel und einem vergrössert wiedergegebenen Ausschnitt daraus.
- Figur 5:: zeigt eine schematische Aufsicht eines Lentikels im Augengewebe, welches mit mehreren konzentrischen, quer zu Meridianen des Lentikels verlaufenden kreisförmigen Bearbeitungslinien und quer dazu verlaufenden Scannlinien geschnitten wird.
- Figur 6:: zeigt eine schematische Aufsicht eines Lentikels im Augengewebe, welches mit mehreren konzentrischen, quer zu Meridianen des Lentikels verlaufenden elliptischen Bearbeitungslinien und quer dazu verlaufenden Scannlinien geschnitten wird.
- Figur 7:: zeigt eine schematische Aufsicht eines Lentikels im Augengewebe, welches mit einer spiralförmigen, quer zu Meridianen des Lentikels verlaufenden Bearbeitungslinie und quer dazu verlaufenden Scannlinien geschnitten wird.
- Figur 8:: zeigt eine schematische Aufsicht eines Lentikels im Augengewebe, welches mit spiralarmförmigen, quer zu Meridianen des Lentikels verlaufenden Bearbeitungslinien und quer dazu verlaufenden Scannlinien geschnitten wird.
- Figur 9:: zeigt eine schematische Aufsicht eines Lentikels im Augengewebe, welches mit mehreren konzentrischen, quer zu Meridianen des Lentikels verlaufenden Bearbeitungslinien und quer dazu verlaufenden Scannlinien geschnitten wird.
- Figur 10:: zeigt eine schematische Aufsicht eines Lentikels im Augengewebe, welches mit mehreren konzentrischen, quer zu Meridianen des Lentikels verlaufenden gekrümmten Bearbeitungslinien und quer dazu verlaufenden Scannlinien geschnitten wird.
- Figur 11:: zeigt eine schematische Querschnittsansicht eines Lentikels, das zur Korrektur von Hyperopie im applanierten Zustand der Cornea in das Augengewebe der Cornea geschnitten wird.
- Figur 12:: zeigt eine schematische Aufsicht eines Lentikels im Augengewebe, welches mit spiralarmförmigen, quer zu Meridianen des Lentikels verlaufenden elliptischen Bearbeitungslinien und quer dazu verlaufenden Scannlinien geschnitten wird.
- Figur 13:: zeigt eine schematische Aufsicht eines Lentikels im Augengewebe, welches mit mehreren parallelen, quer zu Meridianen des Lentikels verlaufenden Bearbeitungslinien und quer dazu verlaufenden Scannlinien geschnitten wird.
- Figur 14:: zeigt eine schematische Querschnittsansicht eines Lentikels, welches mit mehreren parallelen, quer zu Meridianen des Lentikels verlaufenden Bearbeitungslinien und quer dazu verlaufenden Scannlinien geschnitten wird, wobei die Scannlinien nebeneinander verlaufender Bearbeitungslinien unterschiedlich zu einer senkrecht zur Zeichenebene verlaufenden Bearbeitungsebene verkippt sind.
- Figur 15:: zeigt eine schematische Aufsicht von nebeneinander verlaufenden, durch verbleibende Gewebebrücken getrennten Schnittbahnen eines Lentikels im Augengewebe, welche mit einer spiralförmigen elliptischen Bearbeitungslinie und quer dazu verlaufenden Scannlinien geschnitten werden.
- Figur 16:: zeigt eine schematische Aufsicht von sich überlappenden Laserpulsspots eines gepulsten Laserstrahls entlang einer Scannlinie.
- Figur 17:: zeigt ein Flussdiagramm, dass die Durchführung und Erzeugung eines Flächenschnitts im Augengewebe illustriert.
- Figur 18:: zeigt ein Flussdiagramm, dass die Schnittdurchführung und Erzeugung eines Lentikels im Augengewebe illustriert.

### Wege zur Ausführung der Erfindung

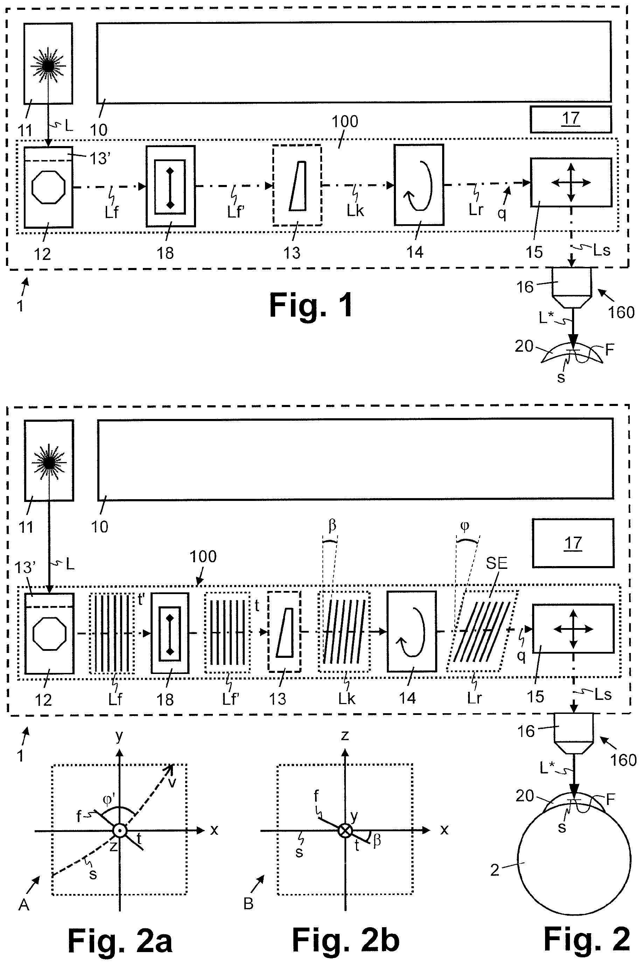

In den Figuren 1 und 2, bezieht sich das Bezugszeichen 1 jeweils auf eine ophthalmologische Vorrichtung zum Bearbeiten von Augengewebe 20 mittels Laserpulsen, beispielsweise die Cornea oder anderes Gewebe eines Auges 2.

Wie in den Figuren 1 und 2 schematisch dargestellt ist, umfasst die ophthalmologische Vorrichtung 1 ein Scannersystem 100, zum Lenken eines von einer Laserquelle 11 gelieferten gepulsten Laserstrahls L über eine Fokussieroptik 16 auf Bearbeitungszielpunkte F im Augengewebe 20, sowie ein Messsystem 17 zur optischen Erfassung von Strukturen im Augengewebe 20. Das Messsystem 17 ist als bildgebendes Messsystem ausgeführt, insbesondere als interferometrisches Messsystem.

Die Fokussieroptik 16 ist eingerichtet zur fokussierten Projektion des gepulsten Laserstrahls L respektive der Laserpulse für die punktuelle Gewebeauflösung in einem Fokus F an einem Bearbeitungszielpunkt im Innern des Augengewebes 20. In den Figuren 1 und 2 wird der von der Fokussieroptik 16 fokussierte Laserstrahl L mit dem Bezugszeichen L* bezeichnet.

Die Fokussieroptik 16 ist beispielsweise in einen Applikationskopf 160 eingebaut, der auf das Auge 2 aufsetzbar ist. Der Applikationskopf 160 wird vorzugsweise über einen mindestens stellenweise lichtdurchlässigen Kontaktkörper oder einer Fluidkammer auf das Auge 2 aufgesetzt und beispielsweise mittels eines vakuumgesteuerten Saugrings am Auge 2 fixiert, wobei der Kontaktkörper und der Saugring fest oder entfernbar mit dem Applikationskopf 160 verbunden sind. Die Fokussieroptik 16 umfasst in einer Ausführungsvariante eine Fokuseinstellvorrichtung zum Einstellen der Fokustiefe, beispielsweise eine oder mehrere bewegliche Linsen, in der Fokussieroptik 16 oder (upstream) der Fokussieroptik 16 vorgeschaltet, oder einen Antrieb zum Bewegen der gesamten Fokussieroptik 16.

Die Laserquelle 11 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 11 ist in einem separaten oder in einem mit der Fokussieroptik 16 gemeinsamen Gehäuse angeordnet.

An dieser Stelle soll festgehalten werden, dass mit dem Bezugszeichen L allgemein der gepulste Laserstrahl L respektive dessen Laserpulse im Strahlengang von der Laserquelle 11 bis zum Fokus F bezeichnet werden, dass jedoch abhängig vom Zusammenhang auch weitere Bezugszeichen verwendet werden, um den gepulsten Laserstrahl L respektive dessen Laserpulse an einer bestimmten Stelle im Strahlengang respektive im Scannersystem 100 zu bezeichnen.

Wie in den Figuren 1 und 2 ersichtlich ist, umfasst das Scannersystem 100 mehrere optische Funktionsmodule, ein erstes Scannermodul 12 (Fast-Scan-Modul), einen Scannlängenmodulator 18, einen z-Modulator 13 respektive 13', einen Rotator 14, und ein zweites Scannermodul 15 (Slow-Scan-Modul). Der Fachmann wird verstehen, dass abhängig von den durch die ophthalmologische Vorrichtung 1 auszuführenden Funktionen, die nachfolgend beschrieben werden, auf den Scannlängenmodulator 18, den z-Modulator 13, 13' und/oder der Rotator 14 verzichtet werden kann. In der ophthalmologischen Vorrichtung 1 gemäss dem zweiten Aspekt der vorliegenden Erfindung ist auch das erste Scannermodul 12 optional.

Wie in den Figuren 1 und 2 schematisch dargestellt ist, umfasst die ophthalmologische Vorrichtung 1 einen Schaltkreis 10 zur Steuerung der Laserquelle 11, der optischen Funktionsmodule des Scannersystems 100, der Fokussieroptik 16 und des Messsystems 17. Der Schaltkreis 10 realisiert eine programmierbare Steuervorrichtung und umfasst beispielsweise ein oder mehrere Prozessoren mit Programm- und Datenspeicher sowie programmierte Softwaremodule zur Steuerung der Prozessoren, und/oder andere programmierbare Schaltungen oder Logikeinheiten wie ASICs (Application Specific Integrated Circuit).

Das der Fokussieroptik 16 vorgelagerte Scannermodul 15 (Slow-Scan-Modul) ist eingerichtet, das Augengewebe mit dem gepulsten Laserstrahl L respektive den Laserpulsen in einer x/y-Bearbeitungsebene entlang einer Bearbeitungslinie s zu scannen, wie beispielhaft in der Aufsicht A der Figur 2a illustriert ist. Das Scannermodul 15 ist als mechanischer Scanner ausgeführt, der die Fokussieroptik 16 mittels einem oder mehreren Bewegungstreibern über ein Bearbeitungsgebiet entlang der Bearbeitungslinie s abfährt, so dass der Fokus F in der x/y-Bearbeitungsebene entlang der Bearbeitungslinie s geführt wird, oder das Scannermodul 15 ist strahlablenkend ausgeführt und umfasst einen oder zwei um jeweils eine oder zwei Achsen bewegliche Ablenkspiegel zum Ablenken des gepulsten Laserstrahls L respektive der Laserpulse in der x/y-Bearbeitungsebene entlang der Bearbeitungslinie s. Das strahlablenkende Scannermodul 15 ist als frei adressierbarer Scanner ausgeführt und umfasst beispielsweise einen Galvanoscanner oder einen Piezo angetriebenen Scanner.

Das dem Scannermodul 15 vorgeschaltete Scannermodul 12 (Fast-Scan-Modul) ist eingerichtet, das Augengewebe mit dem gepulsten Laserstrahl L respektive den Laserpulsen entlang einer der Bearbeitungslinie s überlagerten Scannlinie f zu scannen, wie beispielhaft in der Aufsicht A der Figur 2a illustriert ist. Die beiden Scannersysteme 12 und 15 sind so eingerichtet und gekoppelt, dass die entlang der Scannlinie f verlaufende Scannbewegung der Bearbeitungslinie s des Scannermoduls 15 überlagert ist. Die Scannlinie f verläuft mit einem Ausrichtungswinkel ϕ' quer zur Bearbeitungslinie s. Der Ausrichtungswinkel ϕ' der Scannlinie f zur Bearbeitungslinie s ist durch den Rotator 14 einstellbar. Das Scannermodul 12 umfasst einen oder mehrere bewegliche Ablenkspiegel, beispielsweise ein rotierender Polygonspiegel (Polygonscanner), ein oder mehrere resonante Spiegel (resonanter Scanner) oder oszillierende Spiegel (oszillierender Scanner), die beispielsweise Piezo-angetrieben sind (Piezo-Scanner), oder MEM Scanner (Micro Electro-Mechanical), oder das Scannermodul 12 umfasst einen AOM Scanner (Acusto-optischer Modulator) oder einen EOM Scanner (Electro-optischer Modulator). Das Scannermodul 12 weist eine, beispielsweise mehrfach, höhere Scanngeschwindigkeit auf als das nachgelagerte Scannermodul 15. Entsprechend kann das Scannermodul 12 auch als Fast-Scan-Modul bezeichnet werden, das den abgelenkten Laserstrahl Lf erzeugt, und das Scannermodul 15 kann als Slow-Scan-Modul bezeichnet werden, das den abgelenkten Laserstrahl Ls erzeugt.

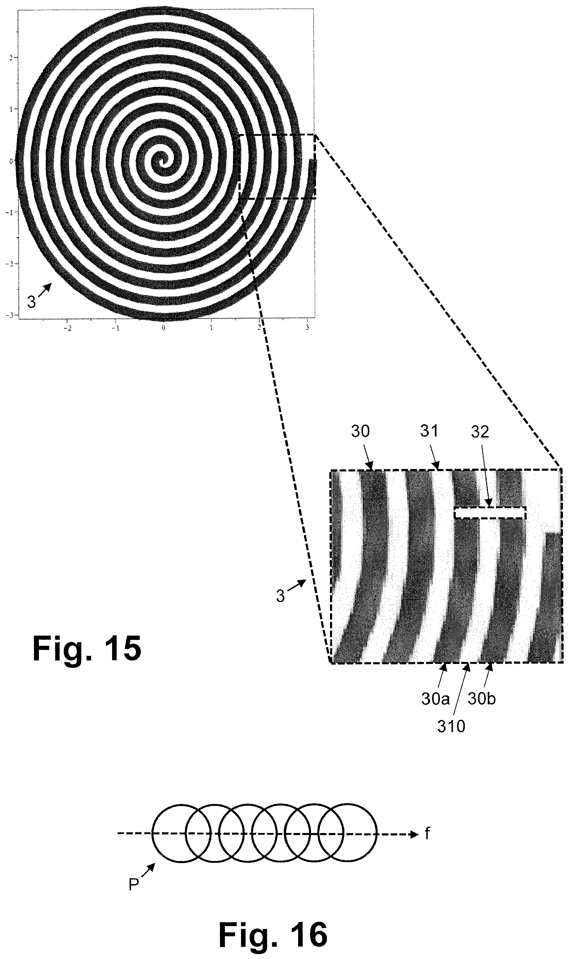

Wie in der Figur 16 illustriert ist, wird der gepulste Laserstrahl vom Scannersystem 100 respektive vom Scannermodul 12 in einer Variante so entlang der Scannlinie f auf Bearbeitungszielpunkte F gelenkt, dass sich die Laserpulsspots P des gepulsten Laserstrahls entlang der Scannlinie f teilweise überlappen, wodurch Gewebebrücken entlang der Scannlinie f verhindert werden. Der Überlappungsgrad ist abhängig von der Scanngeschwindigkeit des Scannermoduls 12 und der Pulsrate der Laserquelle 11 einstellbar.

Wie in den Figuren 1 und 2 schematisch dargestellt ist, ist der Scannlängenmodulator 18 dem Scannermodul 12 nachgeschaltet und eingerichtet, die Länge t der Scannlinie f zu verändern. In einer Ausführungsvariante umfasst der Scannlängenmodulator 18 dazu eine einstellbare Blende, die mit einem ansteuerbaren Antrieb (Elektromotor) gekoppelt ist. Wie in der Figur 2 schematisch dargestellt ist, reduziert der Scannlängenmodulator 18 die Länge t' der Scannlinie f im vom Scannermodul 12 erzeugten abgelenkten Laserstrahl Lf auf die Solllänge t der Scannlinie f im vom Scannermodul 12 abgelenkten und vom Scannlängenmodulator 18 begrenzten Laserstrahl Lf'.

Wie in den Figuren 1 und 2 schematisch dargestellt ist, ist der mit Bezugszeichen 13 bezeichnete z-Modulator dem Scannermodul 12 nachgeschaltet oder in einer mit dem Bezugszeichen 13' bezeichneten alternativen Ausführung dem Scannermodul 12 vorgeschaltet. Der z-Modulator 13 respektive 13' ist eingerichtet, die Scannlinie f aus der x/y-Bearbeitungsebene des Scannermoduls 15 zu verkippen, beispielsweise um den Verkippungswinkel β, wie in der Querschnittsansicht B der Figur 2b schematisch illustriert ist, respektive die Scannlinie f mit einem dynamisch veränderbaren Verkippungswinkel β bezüglich der x/y-Bearbeitungsebene des Scannermoduls 15 zu krümmen. Wie in der Figur 2 schematisch dargestellt ist, erzeugt der z-Modulator 13, 13' einen divergenzmodulierten Laserstrahl Lk mit resultierender Verkippung respektive Verkrümmung der Scannlinie f.

In der dem Scannermodul 12 nachgeschalteten Variante umfasst der z-Modulator 13 eines oder mehrere der Fokussieroptik 16 vorgelagerte optische Elemente, die im Strahlengang vom Scannermodul 12 zur Fokussieroptik 16 angeordnet sind, und die eingerichtet sind im Strahlengang eine vom Scannwinkel des Scannermoduls 12 abhängige Divergenz des Laserstrahls L zu erzeugen. Ausführungen der optischen Elemente des z-Modulators 13 umfassen beispielsweise Keilplatten, Prismen, Linsen, diffraktive optische Elemente und asphärische Spiegel. Die optischen Elemente des z-Modulators 13 sind fest installiert oder in einer Variante zum Einstellen der vom Scannwinkel des Scannermoduls 12 abhängigen Divergenz des Laserstrahls L in den Strahlengang einschiebbar respektive aus dem Strahlengang hinausschiebbar. Als Alternative oder zusätzlich sind die optischen Elemente des z-Modulators 13 zum Einstellen der vom Scannwinkel des Scannermoduls 12 abhängigen Divergenz des Laserstrahls L einstellbar respektive justierbar, beispielsweise durch Rotation der optischen Elemente um die optische Achse q, durch Verkippung der optischen Elemente um eine Drehachse oder durch Verschiebung der optischen Elemente entlang einer zur optischen Achse q verkippten Translationsachse. In einer alternativen Ausführungsvariante ist das optische Element 13 direkt im Scannermodul 12 angeordnet, und beispielsweise als Ablenkspiegel ausgestaltet, der eine veränderliche Oberflächenkrümmung aufweist.

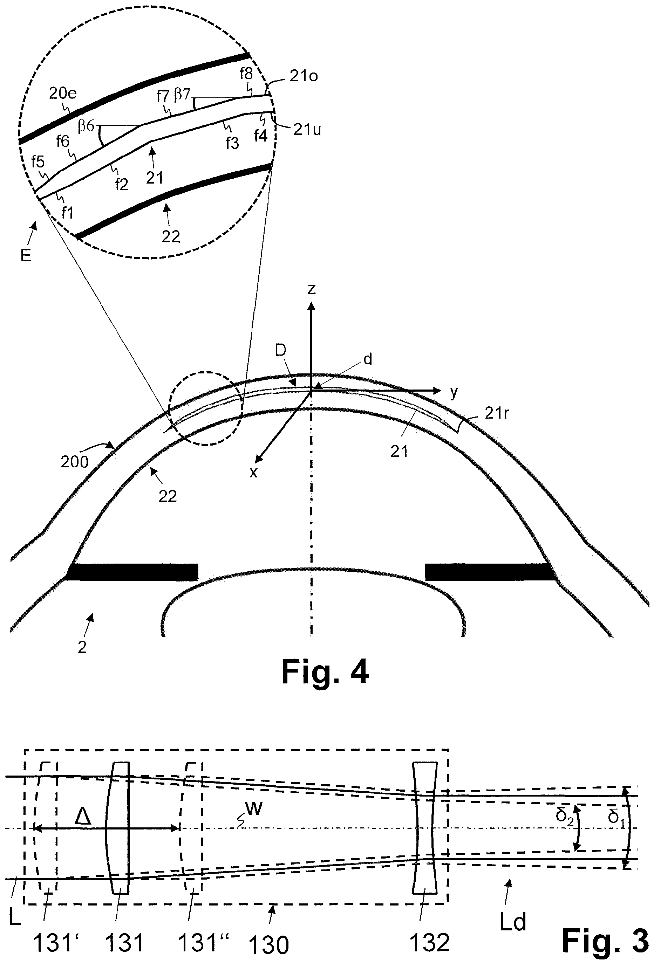

In der dem Scannermodul 12 vorgeschalteten Variante ist der z-Modulator 13' als Divergenzmodulator 130 ausgeführt, der eingerichtet ist, die Divergenz des Laserstrahls L dynamisch zu verändern. Die Figur 3 illustriert schematisch eine Ausführungsvariante des z-Modulators 13' respektive des Divergenzmodulators 130 mit zwei seriell angeordneten optischen Linsen 131, 132, von denen mindestens eine zur Modulation der Divergenz des Laserstrahls L auf einer optischen Übertragungsachse w verschiebbar ist. Zur dynamischen Modulation der Divergenz des Laserstrahls L ist die bewegliche Linse 131 mit einem Bewegungstreiber gekoppelt. Wie im Beispiel der Figur 3 ersichtlich ist, weist der Laserstrahl L bei einer ersten Grundstellung 131' der beweglichen Linse eine entsprechende Divergenz δ₁ auf. Bei einer Verschiebung der beweglichen Linse 131 auf der Übertragungsachse w verändert sich die Divergenz des Laserstrahls L kontinuierlich und hat bei der um die Auslenkungsdistanz Δ verschobenen Stellung 131" eine veränderte Divergenz δ₂.

In alternativen Ausführungen umfasst der z-Modulator 13' einen räumlichen Lichtmodulator zum Modulieren der Wellenfront des Laserstrahls L, ein Flächenlichtmodulator zum Modulieren der Reflexionswinkel an mehreren Punkten einer Reflexionsfläche, über die der Laserstrahl L geleitet wird, ein Brechungsindexmodulator zum Modulieren des Brechungsindexes eines optischen Elements an mehreren Punkten im Querschnitt des Strahlengangs und/oder einen Amplitudenmodulator zur Amplitudenmodulation an mehreren Punkten im Querschnitt des Strahlengangs, also im Strahlprofil, des Laserstrahls L.

In einer weiteren Variante ist der z-Modulator eingerichtet, die Fokussieroptik 16 (einstellbar) um eine Drehachse zu verkippen, die senkrecht zu einer durch die Verarbeitungslinie s und die optische Achse der Fokussieroptik 16 definierten Ebene verläuft, um dadurch die Scannlinie f aus der x/y-Bearbeitungsebene des Scannermoduls 15 um einen einstellbaren Verkippungswinkel zu verkippen.

Durch die Divergenzmodulation verschiebt sich der Fokus F des Laserstrahls L abhängig vom Scannwinkel des Scannermodul 12 in der Projektionsrichtung und erzeugt eine verkippte respektive gekrümmte Scannlinie f. Der z-Modulator 13, 13' respektive Divergenzmodulator 130 ist mit dem Scannermodul 12 so gekoppelt, dass die Veränderung der Divergenz δ₁, δ₂ des Laserstrahls L mit dem Scannwinkel der Scannbewegung synchronisierbar ist, so dass sich eine sich mit dem Scannwinkel des Scannermoduls 12 verändernde, d.h. vom Scannwinkel abhängige Divergenz δ₁, δ₂ des Laserstrahls L ergibt. Der z-Modulator 13, 13' respektive Divergenzmodulator 130 ist eingerichtet, die Divergenz δ₁, δ₂ des Laserstrahls L während der Scannbewegung mit mindestens gleich grosser Frequenz respektive Geschwindigkeit zu modulieren, mit der das Scannermodul 12 die Scannbewegung über den Scannwinkel ausführt um eine Verkippung der Scannlinie f zu bewirken. Um eine "nichtlineare Verkippung" und damit eine Verformung (Verkrümmung) der Scannlinie f in der Projektionsrichtung zu bewirken, ist der z-Modulator 13, 13' respektive Divergenzmodulator 130 eingerichtet, die Divergenz δ₁, δ₂ des Laserstrahls L während der Scannbewegung mit grösseren Frequenzanteilen respektive Geschwindigkeit zu modulieren als das Scannermodul 12 die Scannbewegung über den Scannwinkel ausführt.

Wie in den Figuren 1 und 2 dargestellt ist, ist der Rotator 14 im Strahlengang dem Scannermodul 12 nachgeschaltet und eingerichtet die Fastscannebene Lf, die durch die Scannbewegung des Scannermoduls 12 und die optische Übertragungsachse q definiert ist, um einen Drehwinkel ϕ um die optische Übertragungsachse q zu drehen, so dass eine um den Drehwinkel ϕ rotierte Fastscannebene SE definiert wird, wie in der Figur 2 schematisch dargestellt ist. In der Figur 2 wird der Laserstrahl L mit der vom Rotator 14 gedrehten Fastscannebene mit dem Bezugszeichen Lr bezeichnet. In einer Ausführungsvariante umfasst der Rotator 14 einen K-Spiegel oder ein Prisma zum Drehen der Fastscannebene Lf.

Zum besseren Verständnis der Figur 2 soll hier angemerkt werden, dass in der schematischen Darstellung der rotierten Fastscannebene SE eine Vielzahl von (längenmodulierten, verkippten und rotierten) Scannlinien wiedergegeben ist, die upstream vom Scannermodul 12 durch Ablenkung des gepulsten Laserstrahls L erzeugt werden, in ihrer Länge durch den Scannlängenmodulator 18 auf die Länge t' begrenzt werden, durch den z-Modulator 13, 13' respektive Divergenzmodulator 130 verkippt respektive verkrümmt werden, und schliesslich durch den Rotator 14 um die optische Übertragungsachse q gedreht werden.

Durch Steuerung des Scannermoduls 12 (Fast-Scan-Modul) und des Scannermoduls 15 (Slow-Scan-Modul) wird der gepulste Laserstrahl in Vorschubrichtung v entlang einer Bearbeitungslinie s auf Bearbeitungszielpunkte F im Augengewebe 20 gelenkt und das Augengewebe 20 entlang Scannlinien f bearbeitend gescannt, die der Bearbeitungslinie s überlagert quer zur Bearbeitungslinie s verlaufen. In Kombination mit einer entsprechenden Steuerung der Funktionsmodule des Scannersystems 100 durch den Schaltkreis 10 wird dabei die Scannlinie f durch das Scannlängenmodulator 18 in ihrer Länge t, t', durch den z-Modulator 13, 13' in ihrer Verkippung respektive Verkrümmung gegenüber der x/y-Bearbeitungsebene, und durch der Rotator 14 in ihrer Ausrichtung in der x/y-Bearbeitungsebene zur Bearbeitungslinie s dynamisch eingestellt und verändert, so dass im Augengewebe 20 Flächen in beliebig vorgegebener Freiform geschnitten werden. Durch Schneiden von zwei derartigen Schnittflächen im Augengewebe 20 werden im Augengewebe Lentikel 21 beliebig vorgegebener Freiform geschnitten, wodurch selbst die Korrektur von Aberrationen höherer Ordnung ermöglicht wird.

In den folgenden Abschnitten werden mit Bezug zu den Figuren 4-16 Beispiele möglicher Schnittverfahren und Schnittformen beschrieben, die mit entsprechend eingerichtetem Schaltkreis 10 und damit durchgeführter Steuerung der ophthalmologische Vorrichtung 1 respektive der Funktionsmodule des Scannersystems 100 ausgeführt werden.

Figur 4 illustriert in der Querschnittsansicht, normal zur x/y-Bearbeitungsebene, das Schneiden eines Lentikels 21 in der Cornea 22 eines Auges 2 mittels einer Vielzahl von aneinandergrenzenden Schnittbahnen, die durch bearbeitendes Scannen der Cornea 22 mit dem gepulsten Laserstrahl L auf Bearbeitungszielpunkte F entlang Scannlinien f1, f2, f3, f4, f5, f6, f7, f8 erzeugt werden, wie im vergrössert dargestellten Ausschnitt E ersichtlich ist. Dabei sind die Schnittbahnen respektive die dazu ausgeführten Scannlinien f1, f2, f3, f4, f5, f6, f7, f8 mittels des z-Modulators 13, 13' jeweils um einen unterschiedlichen Verkippungswinkel β5, β6, β7, β8 bezüglich der x/y-Bearbeitungsebene verkippt, wie im vergrössert dargestellten Ausschnitt E für die Schnittbahnen respektive Scannlinien f5, f6, f7, f8 der oberen Aussenfläche 21o des Lentikels explizit angegeben ist, und bei den Schnittbahnen respektive Scannlinien f1, f2, f3, f4 der unteren Aussenfläche 21u des Lentikel 21 ebenfalls ersichtlich ist. Durch die individuell eingestellten Verkippungswinkel β5, β6, β7, β8 der Scannlinien f1, f2, f3, f4, f5, f6, f7, f8 wird der Verlauf möglichst an die Form (Steigung) der Aussenflächen 21o, 21u des zu schneidenden Lentikels 21 angeglichen. Die obere und untere Aussenfläche 21o, 21u treffen sich im peripheren Rand 21r des zu schneidenden Lentikels 21, der in einer Variante als Zylinderfläche ausgeführt wird.

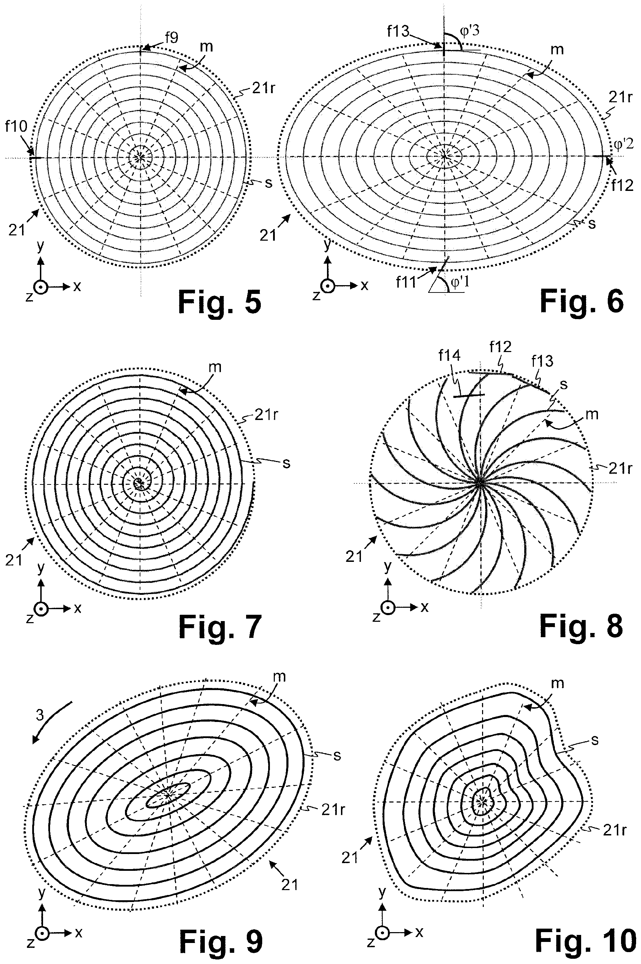

Die Figuren 5, 6, 7, 8, 9 und 10 illustrieren in der Aufsicht auf die x/y-Bearbeitungsebene verschiedene Bearbeitungslinien s zum Schneiden des Lentikels 21 in der Cornea 22 eines Auges 2 mittels Scannlinien f, die den jeweiligen Bearbeitungslinien s überlagert sind und (wie in Figur 2a illustriert) einen Orientierungswinkel ϕ' zur Bearbeitungslinie s aufweisen. Wie in den Figuren 5, 6, 7, 8, 9 und 10 ersichtlich ist, verlaufen die Bearbeitungslinien s quer zu den Meridianen m des Lentikels 21, das heisst die Bearbeitungslinien s verlaufen nicht entlang der Meridiane m des Lentikels 21, sondern schneiden zumindest einen der Meridiane m des Lentikels 21. Die Bearbeitungslinien s gemäss den Figuren 5, 6, 9 und 10 verlaufen jeweils in der x/y-Bearbeitungsebene, zwar auf unterschiedlichen Höhen respektive Tiefen in z-Richtung, aber eine Veränderung in z-Richtung während des Scannens einer geschlossenen Bearbeitungslinie s durch das zweite Scannermodul 15 ist nicht erforderlich. Bei den Bearbeitungslinien s gemäss den Figuren 7, 8, 12 und 13 ist eine Verstellung in z-Richtung während dem Scannen einer Bearbeitungslinie s durch das zweite Scannermodul 15 erforderlich. Abhängig von der Ausführung des zweiten Scannermoduls 15 erfolgt die Anpassung der z-Komponente dieser höhenvariierenden Bearbeitungslinien s an die Aussenfläche 21o, 21u durch entsprechende Steuerung des zweiten Scannermoduls 15 oder einer separaten Fokussiervorrichtung durch den Schaltkreis 10. In einer weiteren Ausführungsvariante ist dazu eine der Fokussieroptik 16 vorgeschaltete und dem z-Modulator 13, 13' vor- oder nachgeschaltete einstellbare Optik vorgesehen (z.B. mit verschiebbaren Linsen), welche eingerichtet ist, die vom z-Modulator 13, 13' verkippte oder verkrümmte Scannlinie f vertikal in z-Richtung zu verschieben. Dabei soll hier aber zum besseren Verständnis angeführt werden, dass solche einstellbaren Optiken nicht die Aufgabe des z-Modulators 13, 13' respektive Divergenzmodulators 130 erfüllen können, da sie einerseits nicht mit dem Scannermodul 12 synchronisiert sind, und sie andererseits eine Fokusverschiebung in z-Richtung auch gar nicht genügend schnell durchführen können, um mit der Scannbewegung respektive dem entsprechenden Scannwinkel des Scannermoduls 12 (Fast-Scan-Modul) synchronisiert werden zu können. Diese einstellbaren Optiken ermöglichen auch keine einstellbare Verkippung der Scannlinie f, wie mit der Variante erreicht wird, in welcher der z-Modulator die Fokussieroptik 16 um eine Drehachse verkippt,

Die Figur 5 zeigt eine Schnittausführung mit mehreren konzentrisch zur optischen Achse des Auges 2 angeordneten, kreisförmigen Bearbeitungslinien s. Die Figur 6 zeigt eine Schnittausführung mit mehreren elliptischen Bearbeitungslinien s, die konzentrisch zur optischen Achse des Auges 2 angeordnet sind. Die Figur 7 zeigt eine Schnittausführung mit einer spiralförmigen Bearbeitungslinie s mit einem Spiralzentrum auf der optischen Achse des Auges 2. Die Figur 8 zeigt eine Schnittausführung mit mehreren spiralarmförmigen Bearbeitungslinien s, die ausgehend vom peripheren Rand 21r des Lentikels 21 auf einen Zentrumspunkt auf der optischen Achse des Auges 2 hinzulaufen (oder umgekehrt). Die Figur 9 zeigt eine Schnittausführung mit mehreren elliptischen Bearbeitungslinien s, die konzentrisch zur optischen Achse des Auges 2 angeordnet sind, wobei die Längs- und Querachsen des Lentikel 21 s im Unterschied zum Lentikel 21 der Figur 6 in der x/y-Bearbeitungsebene um die optische Achse des Auges 2 verdreht ist, wie mit dem Pfeil 3 angedeutet ist. Die Figur 10 zeigt eine Schnittausführung mit mehreren Bearbeitungslinien s, die jeweils durch eine freiförmige geschlossene Kurve definiert sind.

Gegenüber der Schnittausführung gemäss den Figuren 5 und 6, weist die Schnittausführung gemäss der Figur 7 den Vorteil auf, dass sie beim Schneiden eines (dreidimensionalen) Lentikels 21 eine langsame und kontinuierliche Höhenverstellung der Bearbeitungslinie s und damit der dazu quer verlaufenden Scannlinie f erlaubt; das heisst es kann auf sprunghafte und schnellere Änderungen in z-Richtung verzichtet werden.

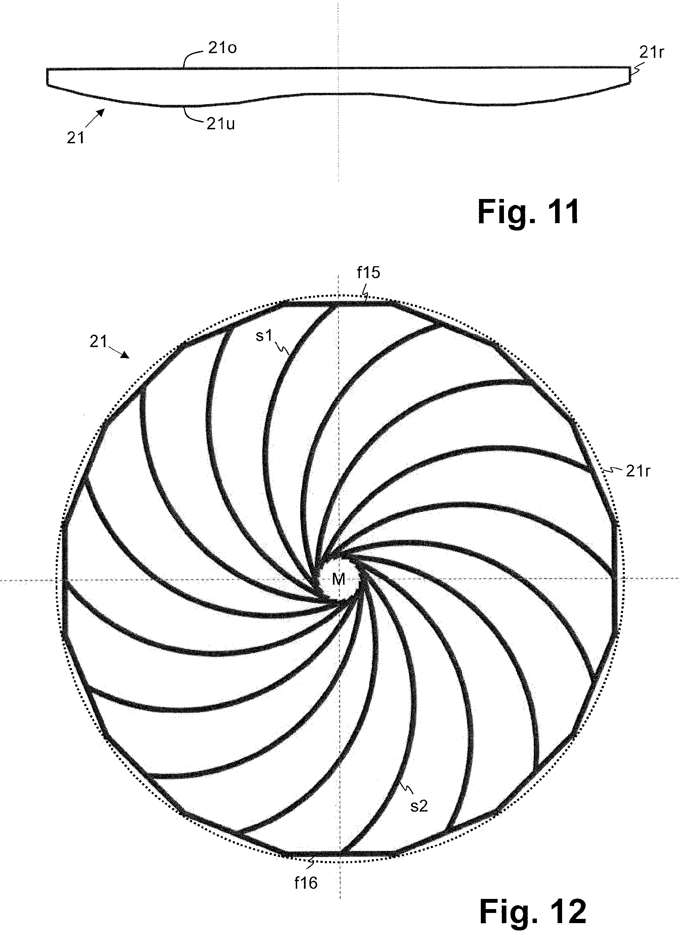

Der Vorteil der Schnittausführung gemäss den Figuren 8 und 12 besteht darin, dass bezüglich der optischen Achse des Auges 2 keine periodischen Strukturen erzeugt werden, die beim Sehen zu störenden Einflüssen auf die Abbildung führen können, da die Drehung der Scannlinie f ein geringeres Mass an bevorzugter (gehäufter) Orientierung (Ausrichtung) aufweist.

Wie in den Figuren 5, 6, 7, 8, 9 und 10 illustriert ist, wird die Ausrichtung respektive der Ausrichtungswinkel ϕ' der Scannlinie f zur Bearbeitungslinie s vom Rotator 14, gesteuert vom Schaltkreis 10, abhängig von der aktuellen Position des Bearbeitungszielpunkts F auf der Bearbeitungslinie s während der Bearbeitung dynamisch verändert und eingestellt. So weisen in der Schnittausführung der Figur 5 die Scannlinie f9, f10 jeweils einen Ausrichtungswinkel ϕ' von 90° also eine normale Ausrichtung zur Bearbeitungslinie s auf, was durch den Rotator 14 durch laufende Veränderung und Anpassung des Drehwinkels ϕ an die Bearbeitungslinie s erreicht wird.

In der Schnittausführung gemäss der Figur 6 ist die Scannlinie 11 mit einem nicht normal zur Bearbeitungslinie s verdrehten Ausrichtungswinkel ϕ'1 zur Bearbeitungslinie s ausgerichtet, so dass, im Vergleich zu einer normal zur Bearbeitungslinie s ausgerichteten Scannlinie f, bei der Bearbeitung im schmäleren Bereich des elliptischen Lentikels 21 eine verschmälerte Bearbeitungsbahn respektive Schnittbahn erzeugt wird. Die Scannlinie f12 ist mit dem Ausrichtungswinkel ϕ'2 normal zur Bearbeitungslinie s ausgerichtet, um im längeren Bereich des elliptischen Lentikels 21 eine breitere Bearbeitungsbahn respektive Schnittbahn zu erzeugen. Die Scannlinie f13 schliesslich ist mit dem Ausrichtungswinkel ϕ'3 normal zur Bearbeitungslinie s ausgerichtet, weist aber im Vergleich zu den Scannlinien f11 und f12 eine durch den Scannlängenmodulator 18 eingestellte kürzere Länge t auf, um bei der Bearbeitung im schmäleren Bereich des elliptischen Lentikels 21 eine verschmälerte Bearbeitungsbahn respektive Schnittbahn zu erzeugen.

Auch in den Schnittausführungen gemäss den Figuren 7 und 9 werden die der spiralförmigen respektive den elliptischen Bearbeitungslinien s überlagerten Scannlinien jeweils hinsichtlich ihres Ausrichtungswinkels ϕ' zur Bearbeitungslinie s, ihrer Länge t und/oder ihres Verkippungswinkels β respektive ihrer Verkrümmung abhängig von der aktuellen Position des Bearbeitungszielpunkts F auf der Bearbeitungslinie s während der Bearbeitung dynamisch verändert und eingestellt, um das Lentikel 21 mit spiralförmigen respektive elliptischen Bearbeitungsbahnen respektive Schnittbahnen zu erzeugen.

In der Schnittausführung gemäss der Figur 8 werden der Ausrichtungswinkels ϕ', die Länge t und der Verkippungswinkel β respektive die Verkrümmung der Scannlinie F12, F13, F14 jeweils abhängig von der aktuellen Position des Bearbeitungszielpunkts F auf dem Spiralarm während der Bearbeitung dynamisch verändert und eingestellt, um das Lentikel 21 mit spiralarmförmigen Bearbeitungsbahnen respektive Schnittbahnen zu erzeugen, deren Breite sich ausgehend vom peripheren Rand 21r des Lentikels 21 zum Zentrumspunkt auf der optischen Achse des Auges 2 hin verkleinert, respektive vom Zentrumspunkt aus zur peripheren Rand 21r wieder vergrössert.

In der Schnittausführung gemäss der Figur 10 werden die den Bearbeitungslinien s überlagerten Scannlinien jeweils hinsichtlich ihres Ausrichtungswinkels ϕ' zur Bearbeitungslinie s, ihrer Länge t und ihres Verkippungswinkels β respektive ihrer Verkrümmung abhängig von der aktuellen Position des Bearbeitungszielpunkts F auf der Bearbeitungslinie s während der Bearbeitung dynamisch verändert und eingestellt, um das Lentikel 21 mit vorgegebener Freiform zur Korrektur einer Aberration höherer Ordnung zu erzeugen.

In der Schnittausführung gemäss der Figur 12 werden der Ausrichtungswinkels ϕ' und die Länge der Scannlinie F12, F13, F14 wie beim Beispiel der Figur 8 jeweils abhängig von der aktuellen Position des Bearbeitungszielpunkts F auf dem Spiralarm s1, s2 während der Bearbeitung dynamisch verändert und eingestellt, um das Lentikel 21 mit spiralarmförmigen Bearbeitungsbahnen respektive Schnittbahnen zu erzeugen, deren Breite sich ausgehend vom peripheren Rand 21r des Lentikels 21 zum Zentrumspunkt auf der optischen Achse des Auges 2 hin verkleinert, respektive vom Zentrumspunkt aus zur peripheren Rand 21r wieder vergrössert. Im Unterschied zu der Ausführung gemäss der Figur 8 werden die Spiralarmförmigen Bearbeitungslinien s1, s2 jedoch nicht bis zum Zentrumspunkt auf der optischen Achse des Auges 2 geführt, sondern einen runden Freiraum M bildend und eine vorgegebene Distanz zum Zentrumspunkt einhaltend um den Zentrumspunkt herumgeführt, so dass ein vom peripheren Rand 21r ausgehender Spiralarm s1 bis zur Grenze des Freiraums M geführt wird und von diesem ausgehend als Spiralarm s2 wieder zum peripheren Rand 21r des Lentikels 21 geführt wird (oder umgekehrt). Dabei wird die Länge der den spiralförmigen Bearbeitungslinien überlagerten Scannlinien an der Grenze des Freiraums M so eingestellt, dass bei der Bearbeitung des Freiraums M durch die Scannlinien möglichst wenig Überschneidungen verursacht werden.

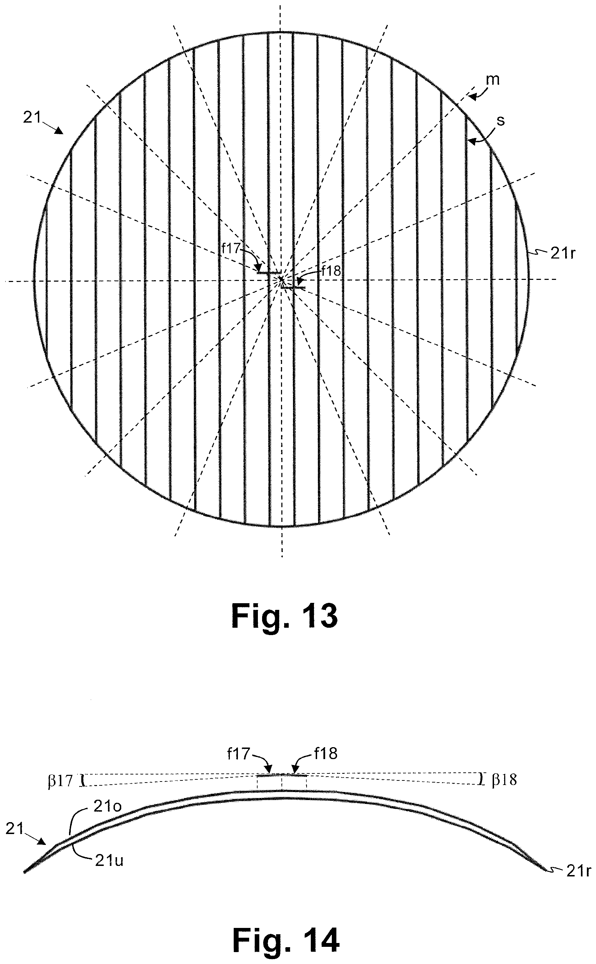

Die Figuren 13 und 14 zeigen eine Schnittausführung mit mehreren Bearbeitungslinien s, die in zur optischen Achse des Auges 2 parallel verlaufenden Ebenen verlaufen und jeweils mehrere Meridiane m des Lentikels 21 schneiden. Die den Bearbeitungslinien s überlagerten Scannlinien f17, f18 sind jeweils mit einem Ausrichtungswinkel ϕ' von 90°, also normal zu der betreffenden Bearbeitungslinie s ausgerichtet. Wie in der Figur 14 im Querschnitt schematisch illustriert ist, sind die Scannlinien f17, f18 jeweils mit einem Verkippungswinkel β17, β18 aus der x/y-Bearbeitungsebene verkippt, um sie an den Verlauf der zu schneidenden oberen respektive unteren Aussenfläche 21o, 21u des Lentikels 21 anzupassen.

Die Figur 11 zeigt im Querschnitt eine Schnittausführung eines Lentikels 21 mit oberer und unterer Aussenfläche 21o, 21u und einem peripheren Rand 21r, welche im applanierten Zustand der Cornea zur Korrektur von Hyperopie in das Augengewebe der Cornea 2 geschnitten werden. Die obere und untere Aussenfläche 21o, 21u des Lentikels 21 werden mit einer Schnittausführungen gemäss einer der Figuren 5, 6, 7, 8, 9, 10, 12 oder 14 geschnitten. Der periphere Rand 21r wird mit einer um 90° aus der x/y-Bearbeitungsebene verkippten Scannlinie geschnitten, die einer das zu schneidende Lentikel 21 im peripheren Rand 21r umlaufenden Bearbeitungslinie s überlagert ist.

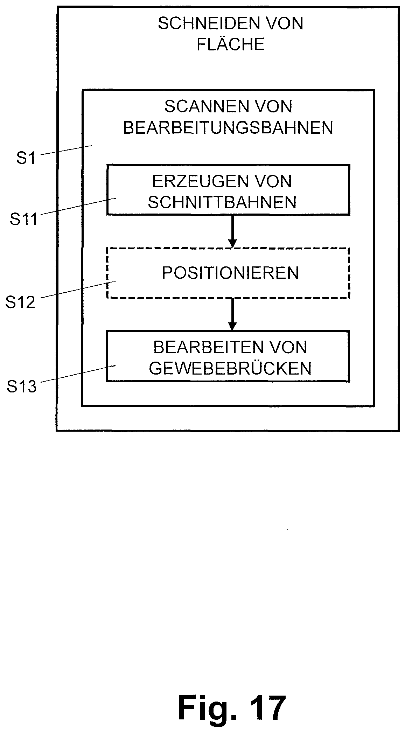

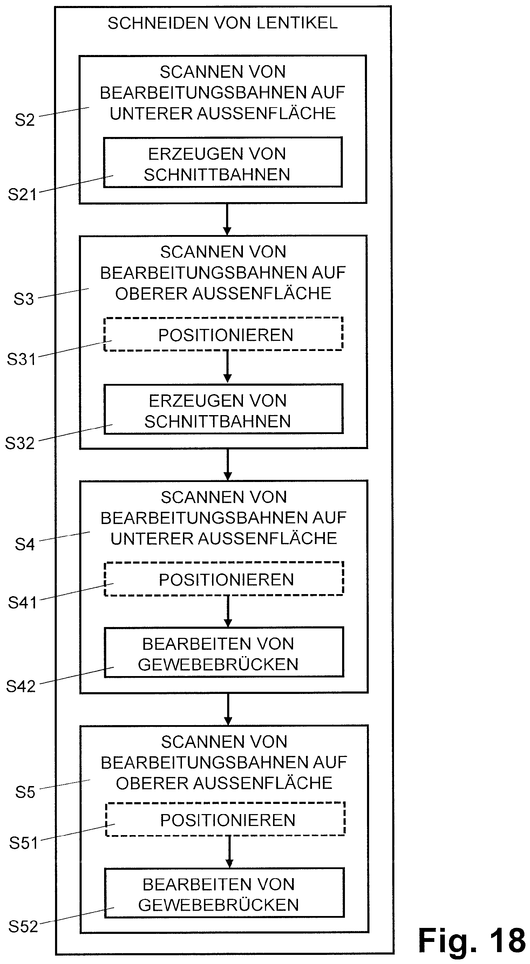

An dieser Stelle soll zum besseren Verständnis festgehalten werden, dass auch die obere und untere Aussenfläche 21o, 21u des in der Figur 4 im Querschnitt dargestellten Lentikels 21 im nicht applanierten Zustand der Cornea 2 mit einer Schnittausführung gemäss einer der Figuren 5, 6, 7, 8, 9, 10, 12 oder 13/14 geschnitten wird. Dasselbe gilt für die nachfolgend erläuterten Schnittverfahren gemäss den Figuren 17 und 18.

In den folgenden Abschnitten wird mit Bezug zu den Figuren 17 und 18 die vom Schaltkreis 10 gesteuerte Durchführung und Erzeugung eines Flächenschnitts im Augengewebe 20 und die vom Schaltkreis 10 gesteuerte Schnittdurchführung und Erzeugung eines Lentikels im Augengewebe 20 illustriert.

Zum Schneiden einer Fläche im Augengewebe 20 steuert der Schaltkreis 10 das Scannersystem 100 respektive dessen optische Funktionsmodule um den gepulsten Laserstrahl auf Bearbeitungszielpunkte F im Augengewebe 20 zu lenken. Zum Erzeugen der Schnittfläche steuert der Schaltkreis 10 das Scannersystem 100 im Schritt S1 derart, dass der gepulste Laserstrahl das Augengewebe 20 in Bearbeitungsbahnen scannt, die parallel nebeneinander verlaufen oder die nebeneinander verlaufen und eine spiralförmige, kreisförmige oder elliptische Form aufweisen. Dabei lenkt das zweite Scannermodul 15 des Scannersystems 100 den gepulsten Laserstrahl in Vorschubrichtung v auf Bearbeitungszielpunkte F entlang einer Bearbeitungslinie s und das erste Scannermodul 12 des Scannersystems 100 lenkt den gepulsten Laserstrahl auf Bearbeitungszielpunkte F entlang einer quer zur Bearbeitungslinie s verlaufenden Scannlinie f ab, wie in den Figuren 2a, 2b und 4-14 illustriert und obenstehend mit Bezug zu diesen Figuren beschrieben wurde, so dass im Schritt S11 eine durch die Bearbeitungsbahn definierte Schnittbahn erzeugt wird.

Im Schritt S11 steuert der Schaltkreis 10 das Scannersystem 100 respektive dessen optische Funktionsmodule so, dass die Schnittbahnen in mehreren Bearbeitungsbahnen erzeugt werden, wobei jeweils verbleibende Gewebebrücken zwischen den Schnittbahnen belassen werden. Dies ist in Figur 15 am Beispiel von spiralförmigen Bearbeitungslinien respektive dadurch definierten spiralförmigen Bearbeitungsbahnen und resultierender Schnittbahn 30 illustriert. Im oberen Teil der Figur 15 sind die nebeneinander verlaufenden Bearbeitungsbahnen 3 ersichtlich. Wie im unteren Teil der Figur 15 in einem vergrössert dargestellten Ausschnitt ersichtlich ist, werden die nebeneinander verlaufenden Bearbeitungsbahnen 3 im Schritt S11 so bearbeitet, dass in den bearbeiteten Bearbeitungsbahnen Schnittbahnen 30 erzeugt werden, die durch Gewebebrücken 31 in dazwischenliegenden, noch unbearbeiteten Bearbeitungsbahnen getrennt sind. Abhängig von der Form der Bearbeitungslinie s sind die darauf basierenden Bearbeitungsbahnen 3 respektive Schnittbahnen 30 parallel nebeneinander verlaufend oder verlaufen spiralförmig, kreisförmig, elliptisch oder in freiförmig geschlossenen Kurven nebeneinander.

Im optionalen Schritt S12, steuert der Schaltkreis 10 das Messsystem 17 derart, dass dieses die erzeugten Schnittbahnen 30 erfasst, und die verbliebenen Gewebebrücken 31 basierend auf den erzeugten Schnittbahnen 30 bestimmt. Aufgrund der bestimmten Gewebebrücken 31 bestimmt der Schaltkreis 10 die unbehandelten noch zu behandelnden Bearbeitungsbahnen. In der Figur 15 bezieht sich das Bezugszeichen 32 auf einen erfassten Ausschnitt der erzeugten Schnittbahnen 30a, 30b zur Bestimmung der dazwischenliegenden Gewebebrücke 310 und der noch zu bearbeitenden entsprechenden Bearbeitungsbahn. Insbesondere im Falle des als interferometrisches Messsystem ausgeführten Messsystems 17 erfolgt die Erfassung der erzeugten Schnittbahnen 30a, 30b und die Bestimmung der dazwischenliegenden Gewebebrücke 310 zur Positionierung der zu bearbeitenden Bearbeitungsbahn laufend während der Bearbeitung ("online"), so dass der aktuelle Bearbeitungszielpunkt F der Bearbeitungslinie s respektive der quer dazu ausgerichteten Scannlinie f in Bearbeitungsrichtung v dem erfassten Ausschnitt 32 ("downstream") der erzeugten Schnittbahnen 30a, 30b hinterhergehend nachfolgt ("upstream").

Im Schritt S13 steuert der Schaltkreis 10 das Scannersystem 100 respektive dessen optische Funktionsmodule so, dass die bestimmten Gewebebrücken 31 respektive noch unbehandelten Bearbeitungsbahnen bearbeitet werden. Mit anderen Worten, das Scannersystem 100 wird so gesteuert, dass es den gepulsten Laserstrahl auf Bearbeitungszielpunkte F in die zwischen den Schnittbahnen 30 verbliebenen Gewebebrücken 31 lenkt, um den Gewebeschnitt zu vervollständigen. In einer Ausführungsvariante werden die Gewebebrücken in Bearbeitungsbahnen 3 bearbeitet, deren Breite über die Breite der bestimmten Gewebebrücken 31 hinausgeht. Wenn sämtliche verbliebenen Gewebebrücken 31 bearbeitet sind, ist die Fläche vollständig geschnitten und die entsprechende Schnittfläche im Augengewebe 20 erzeugt.

Zum Schneiden eines Lentikels 21 im Augengewebe werden zwei Schnittflächen, die untere Aussenfläche 21u und die obere Aussenfläche 21o des Lentikels 21 geschnitten.

Die Figur 18 illustriert ein Schnittverfahren, mit welchem in einer Sequenz von Schnittfolgen ein Lentikel 21 im Augengewebe 20 geschnitten wird. Dabei steuert der Schaltkreis 10 das Scannersystem 100 respektive dessen optische Funktionsmodule so, dass im Schritt S2 die Bearbeitungsbahnen 30 auf der unteren Aussenfläche 21u des zu schneidenden Lentikels 21 bearbeitet und im Schritt S21 die Schnittbahnen 30 mit verbleibenden Gewebebrücken 31 erzeugt werden, wie obenstehend mit Bezug zur Figur 17 im Zusammenhang mit den Schritten S1 und S11 beschrieben wurde. Mit anderen Worten die untere Aussenfläche 21u des Lentikels 21 wird in den Schritten S2, S21 zunächst unvollständig mit verbleibenden Gewebebrücken 31 zwischen den Schnittbahnen 30 geschnitten.

Im darauffolgenden Schritt S3 wird nun im Unterschied zum Schneiden der Schnittfläche gemäss der Figur 17 damit begonnen, vorher die obere Aussenfläche 21o des Lentikels 21 zu schneiden. Dabei steuert der Schaltkreis 10 das Scannersystem 100 respektive dessen optische Funktionsmodule so, dass im Schritt S3 die Bearbeitungsbahnen 30 auf der oberen Aussenfläche 21o des zu schneidenden Lentikels 21 bearbeitet werden. Dazu steuert der Schaltkreis 10 im optionalen Schritt S31, das Messsystem 17 derart, dass dieses die im Schritt S2 respektive S21 erzeugten Schnittbahnen 30 auf der unteren Aussenfläche 21u erfasst, und die zu bearbeitenden Bearbeitungsbahnen 3 respektive die zu schneidenden Schnittbahnen 30 auf der oberen Aussenfläche 21o basierend auf den erzeugten Schnittbahnen 30 der unteren Aussenfläche 21u bestimmt. Dabei wird neben der lateralen Positionierung der oberen Aussenfläche 21o zur unteren Aussenfläche 21u auch eine vorgegebenen Zentrumsdicke d respektive ein vorgegebenes Dickenprofil D des zu schneidenden Lentikels 21 eingehalten. In einer Ausführungsvariante steuert der Schaltkreis 10 das Messsystem 17 zudem so, dass das Messsystem 17 Verformungen im Augengewebe erfasst, die aufgrund von beim Schneiden der Schnittbahnen 30 auf der unteren Aussenfläche 21u erzeugten Gasblasen bewirkt wurden, und die obere Aussenfläche 21o, respektive die zu bearbeitenden Bearbeitungsbahnen 3 und dadurch zu schneidenden Schnittbahnen 30 auf der oberen Aussenfläche 21o, werden unter Berücksichtigung der erfassten Verformungen bezüglich der erfassten unteren Aussenfläche 21u positioniert. Der Schaltkreis 10 steuert dann das Scannersystem 100 respektive dessen optische Funktionsmodule so, dass im Schritt S32 die Schnittbahnen 30 mit verbleibenden Gewebebrücken 31 auf der oberen Aussenfläche 21o erzeugt werden, wie obenstehend mit Bezug zur Figur 17 im Zusammenhang mit den Schritten S1 und S11 beschrieben wurde. In einer Ausführungsvariante bestimmt der Schaltkreis 10 die Bearbeitungsbahnen 3 für die Erzeugung der Schnittbahnen 30 auf der oberen Aussenfläche 21o mit einer kleineren Bahnbreite bezüglich der erfassten Schnittbahnen 30 auf der unteren Aussenfläche 21u. Im Schritt S32 wird auch die obere Aussenfläche 21o des Lentikels 21 unvollständig mit verbleibenden Gewebebrücken 31 zwischen den Schnittbahnen 30 geschnitten.

Im darauffolgenden Schritt S4 werden die auf der unteren Aussenfläche 21u zwischen den Schnittbahnen 30 verbliebenen Gewebebrücken 31 geschnitten. Dazu steuert der Schaltkreis 10 das Scannersystem 100 respektive dessen optische Funktionsmodule so, dass im Schritt S4 die Bearbeitungsbahnen 30 mit den verbliebenen Gewebebrücken 31 auf der unteren Aussenfläche 21u des zu schneidenden Lentikels 21 bearbeitet werden. Im optionalen Schritt S41, steuert der Schaltkreis 10 das Messsystem 17 derart, dass dieses die bereits erzeugten Schnittbahnen 30 auf der unteren Aussenfläche 21u erfasst, und die verbliebenen Gewebebrücken 31 basierend auf den erfassten Schnittbahnen 30 bestimmt. Aufgrund der bestimmten Gewebebrücken 31 bestimmt der Schaltkreis 10 die unbehandelten noch zu behandelnden Bearbeitungsbahnen auf der unteren Aussenfläche 21u. Im Schritt S42 steuert der Schaltkreis 10 das Scannersystem 100 respektive dessen optische Funktionsmodule so, dass die verbliebenen Gewebebrücken 31 respektive noch unbehandelten Bearbeitungsbahnen bearbeitet und dadurch geschnitten werden, wie obenstehend mit Bezug zur Figur 17 im Zusammenhang mit Schritt S13 beschrieben wurde, und vervollständigt dadurch den Gewebeschnitt auf der unteren Aussenfläche 21u des Lentikels 21.

Im darauffolgenden Schritt S5 werden die auf der oberen Aussenfläche 21o zwischen den Schnittbahnen 30 verbliebenen Gewebebrücken 31 geschnitten. Dazu steuert der Schaltkreis 10 das Scannersystem 100 respektive dessen optische Funktionsmodule so, dass im Schritt S5 die Bearbeitungsbahnen 30 mit den verbliebenen Gewebebrücken 31 auf der oberen Aussenfläche 21o des zu schneidenden Lentikels 21 bearbeitet werden. Im optionalen Schritt S51, steuert der Schaltkreis 10 das Messsystem 17 derart, dass dieses die bereits erzeugten Schnittbahnen 30 auf der oberen Aussenfläche 21o erfasst, und die verbliebenen Gewebebrücken 31 basierend auf den erfassten Schnittbahnen 30 bestimmt. Aufgrund der bestimmten Gewebebrücken 31 bestimmt der Schaltkreis 10 die unbehandelten noch zu behandelnden Bearbeitungsbahnen auf der oberen Aussenfläche 21o. Im Schritt S52 steuert der Schaltkreis 10 das Scannersystem 100 respektive dessen optische Funktionsmodule so, dass die verbliebenen Gewebebrücken 31 respektive noch unbehandelten Bearbeitungsbahnen bearbeitet werden, wie obenstehend mit Bezug zur Figur 17 im Zusammenhang mit Schritt S13 beschrieben wurde, und vervollständigt dadurch den Gewebeschnitt auf der oberen Aussenfläche 21o des Lentikels 21, womit das Lentikel 21 vollständig geschnitten ist.

## Patentansprüche

1. Ophthalmologische Vorrichtung (1) zum Bearbeiten von Augengewebe (20), umfassend eine Laserquelle (11), die eingerichtet ist, einen gepulsten Laserstrahl (L) zu erzeugen; eine Fokussieroptik (16), die eigerichtet ist, den gepulsten Laserstrahl ins Augengewebe (20) zu fokussieren; ein Scannersystem (100), das eingerichtet ist, den gepulsten Laserstrahl auf Bearbeitungszielpunkte (F) im Augengewebe (20) zu lenken; ein Messsystem (17), das eingerichtet ist, Strukturen im Augengewebe (20) optisch zu erfassen; und einen Schaltkreis (10), der eingerichtet ist, das Scannersystem (100) derart zu steuern, dass das Scannersystem (100) den gepulsten Laserstrahl auf Bearbeitungszielpunkte (F) auf einer ersten Aussenfläche (21u) eines im Augengewebe (20) zu schneidenden Lentikels (21) lenkt, um einen ersten Gewebeschnitt zum Schneiden des Lentikels (21) zu erzeugen; **dadurch gekennzeichnet,**
**dass** der Schaltkreis (10) überdies eingerichtet ist, das Messsystem (17) derart zu steuern, dass das Messsystem (17) die durch den ersten Gewebeschnitt erzeugte erste Aussenfläche (21u) des Lentikels (21) erfasst, und das Scannersystem (100) derart zu steuern, dass das Scannersystem (100) den gepulsten Laserstrahl auf Bearbeitungszielpunkte (F) auf einer bezüglich der erfassten ersten Aussenfläche (21u) des Lentikels (21) positionierten zweiten Aussenfläche (21o) des zu schneidenden Lentikels (21) lenkt, um einen bezüglich der erfassten ersten Aussenfläche (21u) des Lentikels (21) positionierten zweiten Gewebeschnitt zum Schneiden des Lentikels (21) zu erzeugen.

2. Ophthalmologische Vorrichtung (1) nach Anspruch 1, **dadurch gekennzeichnet, dass** der Schaltkreis (10) eingerichtet ist, die zweite Aussenfläche (21o) des zu schneidenden Lentikels (21) mit einer vorgegebenen Zentrumsdicke (d) des zu schneidenden Lentikels (21) bezüglich der erfassten ersten Aussenfläche (21u) zu positionieren.

3. Ophthalmologische Vorrichtung (1) nach einem der Ansprüche 1 oder 2, **dadurch gekennzeichnet, dass** der Schaltkreis (10) eingerichtet ist, die zweite Aussenfläche des zu schneidenden Lentikels (21) mit einem vorgegebenen Dickenprofil (D) des zu schneidenden Lentikels (21) bezüglich der erfassten ersten Aussenfläche (21u) zu positionieren.

4. Ophthalmologische Vorrichtung (1) nach einem der Ansprüche 1 bis 3, **dadurch gekennzeichnet, dass** der Schaltkreis (10) eingerichtet ist, das Messsystem (17) derart zu steuern, dass das Messsystem (17) durch beim ersten Gewebeschnitt erzeugte Gasblasen bewirkte Verformungen der ersten Aussenfläche (21u) erfasst, und die zweite Aussenfläche (21o) des zu schneidenden Lentikels (21) unter Berücksichtigung der erfassten Verformungen bezüglich der erfassten ersten Aussenfläche (21u) positioniert.

5. Ophthalmologische Vorrichtung (1) nach einem der Ansprüche 1 bis 4, **dadurch gekennzeichnet, dass** der Schaltkreis (10) eingerichtet ist, die erste Aussenfläche (21u) des zu schneidenden Lentikels (21) als eine von einer äusseren Hornhautoberfläche (200) abgewandte Unterseite des zu schneidenden Lentikels (21) zu bestimmen, und die zweite Aussenfläche (21o) des zu schneidenden Lentikels (21) als eine von der äusseren Hornhautoberfläche (200) zugewandte Oberseite des zu schneidenden Lentikels (21) zu bestimmen.

6. Ophthalmologische Vorrichtung (1) nach einem der Ansprüche 1 bis 5, **dadurch gekennzeichnet, dass** der Schaltkreis (10) eingerichtet ist, das Scannersystem (100) derart zu steuern, dass das Scannersystem (100) den gepulsten Laserstrahl auf Bearbeitungszielpunkte (F) in nebeneinander verlaufenden Bearbeitungsbahnen (3) auf der ersten Aussenfläche (21u) des zu schneidenden Lentikels (21) lenkt, um nebeneinander verlaufende Schnittbahnen (30) des ersten Gewebeschnitts zu erzeugen; und das Messsystem (17) derart zu steuern, dass das Messsystem (17) die nebeneinander verlaufenden Schnittbahnen (30) erfasst und die zweite Aussenfläche (21o) des zu schneidenden Lentikels (21) bezüglich der erfassten nebeneinander verlaufenden Schnittbahnen (30) positioniert.

7. Ophthalmologische Vorrichtung (1) nach Anspruch 6, **dadurch gekennzeichnet, dass** der Schaltkreis (10) eingerichtet ist, das Scannersystem (100) derart zu steuern, dass das Scannersystem (100) den gepulsten Laserstrahl auf Bearbeitungszielpunkte (F) in nebeneinander verlaufenden Bearbeitungsbahnen (3) auf der ersten Aussenfläche (21u) des zu schneidenden Lentikels (21) lenkt, um nebeneinander verlaufende, durch verbleibende Gewebebrücken (31) getrennte Schnittbahnen (30) des ersten Gewebeschnitts zu erzeugen; das Messsystem (17) derart zu steuern, dass das Messsystem (17) die nebeneinander verlaufenden Schnittbahnen (30) erfasst; und das Scannersystem (100) derart zu steuern, dass das Scannersystem (100) den gepulsten Laserstrahl auf Bearbeitungszielpunkte (F) in die basierend auf den erfassten nebeneinander verlaufenden Schnittbahnen (30) bestimmten verbleibenden Gewebebrücken (31) auf der ersten Aussenfläche (21u) lenkt, um den ersten Gewebeschnitt zu erzeugen.

8. Ophthalmologische Vorrichtung (1) nach einem der Ansprüche 6 oder 7, **dadurch gekennzeichnet, dass** der Schaltkreis (10) eingerichtet ist, das Scannersystem (100) derart zu steuern, dass das Scannersystem (100) den gepulsten Laserstrahl auf Bearbeitungszielpunkte (F) in Bearbeitungsbahnen (3) lenkt, die auf der Aussenfläche (21o, 21u) des Lentikels (21) (21) verlaufen und eine der folgenden Ausgestaltungen aufweisen: gradlinig, entlang von Kreisen, konzentrisch kreisförmig, konzentrisch elliptisch, spiralförmig und spiralarmförmig.

9. Ophthalmologische Vorrichtung (1) nach einem der Ansprüche 1 bis 8, **dadurch gekennzeichnet, dass** der Schaltkreis (10) eingerichtet ist, ein zweites Scannermodul (15) des Scannersystems (15) derart zu steuern, dass das zweite Scannermodul (15) den gepulsten Laserstrahl auf Bearbeitungszielpunkte (F) in einer Vorschubrichtung (v) entlang einer Bearbeitungslinie (s) lenkt, die auf der ersten respektive zweiten Aussenfläche (21u, 21o) des zu schneidenden Lentikels (21) verläuft; dass das Scannersystem ein erstes Scannermodul (12) umfasst, das eingerichtet ist, den gepulsten Laserstrahl entlang einer um einen Ausrichtungswinkel (δ) quer zur Bearbeitungslinie (s) in einer horizontalen Bearbeitungsebene (x/y) verlaufenden Scannlinie (f) zu lenken, mit einer wesentlich höheren Scanngeschwindigkeit im Vergleich zur Scanngeschwindigkeit des zweiten Scannermoduls (15) in Vorschubrichtung (v); und dass das Scannersystem einen z-Modulator (13) umfasst, der eingerichtet ist, die Scannlinie (f) abhängig von einem bestimmten Bearbeitungszielpunkt des zweiten Scannermoduls (15) auf der Bearbeitungslinie (s) derart aus der Bearbeitungsebene (x/y) zu verkippen, dass die Scannlinie (f) im Wesentlichen entlang der Aussenfläche (21o, 21u) des Lentikels (21) verläuft.

10. Ophthalmologische Vorrichtung (1) nach einem der Ansprüche 1 bis 9, **dadurch gekennzeichnet, dass** das Messsystem (17) als interferometrisches Messsystem ausgeführt ist.

## Claims

1. Ophthalmological apparatus (1) for working on eye tissue (20), including a laser source (11) that is configured to produce a pulsed laser beam (L); a focusing optical unit (16) that is configured to focus the pulsed laser beam into the eye tissue (20); a scanner system (100) that is configured to steer the pulsed laser beam onto work target points (F) in the eye tissue (20); a measurement system (17) that is configured to optically capture structures in the eye tissue (20); and a circuit (10) that is configured to control the scanner system (100) in such a way that the scanner system (100) steers the pulsed laser beam onto work target points (F) on a first outer face (21u) of a lenticule (21) to be cut in the eye tissue (20) in order to produce a first tissue cut for cutting the lenticule (21); **characterized**
**in that** the circuit (10) moreover is configured to control the measurement system (17) in such a way that the measurement system (17) captures the first outer face (21u) of the lenticule (21) that is produced by the first tissue cut and to control the scanner system (100) in such a way that the scanner system (100) steers the pulsed laser beam onto work target points (F) on a second outer face (21o), positioned in relation to the captured first outer face (21u) of the lenticule (21), of the lenticule (21) to be cut in order to produce a second tissue cut, positioned in relation to the captured first outer face (21u) of the lenticule (21), for cutting the lenticule (21).

2. Ophthalmological apparatus (1) according to Claim 1, **characterized in that** the circuit (10) is configured to position the second outer face (21o) of the lenticule (21) to be cut, with a predetermined center thickness (d) of the lenticule (21) to be cut, in relation to the captured first outer face (21u).

3. Ophthalmological apparatus (1) according to either of Claims 1 and 2, **characterized in that** the circuit (10) is configured to position the second outer face of the lenticule (21) to be cut, with a predetermined thickness profile (D) of the lenticule (21) to be cut, in relation to the captured first outer face (21u).

4. Ophthalmological apparatus (1) according to any one of Claims 1 to 3, **characterized in that** the circuit (10) is configured to control the measurement system (17) in such a way that the measurement system (17) captures deformations, caused by gas bubbles produced during the first tissue cut, of the first outer face (21u) and positions the second outer face (21o) of the lenticule (21) to be cut taking account of the captured deformations with respect to the captured first outer face (21u).

5. Ophthalmological apparatus (1) according to any one of Claims 1 to 4, **characterized in that** the circuit (10) is configured to determine the first outer face (21u) of the lenticule (21) to be cut as a lower side of the lenticule (21) to be cut facing away from an outer corneal surface (200) and to determine the second outer face (21o) of the lenticule (21) to be cut as an upper side of the lenticule (21) to be cut facing the outer corneal surface (200).

6. Ophthalmological apparatus (1) according to any one of Claims 1 to 5, **characterized in that** the circuit (10) is configured to control the scanner system (100) in such a way that the scanner system (100) steers the pulsed laser beam onto work target points (F) in work trajectories (3) extending next to one another on the first outer face (21u) of the lenticule (21) to be cut in order to produce cut trajectories (30) of the first tissue cut extending next to one another; and to control the measurement system (17) in such a way that the measurement system (17) captures the cut trajectories (30) extending next to one another and positions the second outer face (21o) of the lenticule (21) to be cut in relation to the captured cut trajectories (30) extending next to one another.

7. Ophthalmological apparatus (1) according to Claim 6, **characterized in that** the circuit (10) is configured to control the scanner system (100) in such a way that the scanner system (100) steers the pulsed laser beam onto work target points (F) in work trajectories (3) extending next to one another on the first outer face (21u) of the lenticule (21) to be cut in order to produce cut trajectories (30) of the first tissue cut that extend next to one another and that are separated by remaining tissue bridges (31); to control the measurement system (17) in such a way that the measurement system (17) captures the cut trajectories (30) that extend next to one another; and to control the scanner system (100) in such a way that the scanner system (100) steers the pulsed laser beam onto work target points (F) in the remaining tissue bridges (31), determined on the basis of the captured cut trajectories (30) that extend next to one another, on the first outer face (21u) in order to produce the first tissue cut.

8. Ophthalmological apparatus (1) according to either of Claims 6 and 7, **characterized in that** the circuit (10) is configured to control the scanner system (100) in such a way that the scanner system (100) steers the pulsed laser beam onto work target points (F) in work trajectories (3) that extend on the outer face (21o, 21u) of the lenticule (21) and that have one of the following configurations: straight lined, along circles, concentrically circular, concentrically elliptical, spiral-shaped and spiral-arm-shaped.

9. Ophthalmological apparatus (1) according to any one of Claims 1 to 8, **characterized in that** the circuit (10) is configured to control a second scanner module (15) of the scanner system (15) in such a way that the second scanner module (15) steers the pulsed laser beam onto work target points (F) in a feed direction (v) along a work line (s) that extends on the first or second outer face (21u, 21o) of the lenticule (21) to be cut; that the scanner system includes a first scanner module (12) that is configured to steer the pulsed laser beam along a scan line (f) extending across the work line (s) at an alignment angle (δ) in a horizontal work plane (x/y), with a substantially higher scan speed in comparison with the scan speed of the second scanner module (15) in the feed direction (v); and **in that** the scanner system includes a z-modulator (13) that is configured to tilt the scan line (f) out of the work plane (x/y) depending on a specific work target point of the second scanner module (15) on the work line (s) in such a way that the scan line (f) extends substantially along the outer face (21o, 21u) of the lenticule (21).

10. Ophthalmological apparatus (1) according to any one of Claims 1 to 9, **characterized in that** the measurement system (17) is embodied as an interferometric measurement system.

## Revendications

1. Dispositif ophtalmologique (1) destiné au traitement de tissu oculaire (20), comprenant une source laser (11) conçue pour générer un faisceau laser pulsé (L) ; une optique de focalisation (16) conçue pour focaliser le faisceau laser pulsé dans le tissu oculaire (20) ; un système de balayage (100) conçu pour diriger le faisceau laser pulsé vers des points cibles de traitement (F) dans le tissu oculaire (20) ; un système de mesure (17) conçu pour détecter optiquement des structures dans le tissu oculaire (20) ; et un circuit (10) conçu pour commander le système de balayage (100) de manière à ce que le système de balayage (100) dirige le faisceau laser pulsé vers des points cibles de traitement (F) sur une première surface extérieure (21u) d'une lenticule (21), destinée à être découpée dans le tissu oculaire (20), afin de produire une première découpe de tissu pour découper la lenticule (21) ; **caractérisé en ce que**
le circuit (10) est en outre conçu pour commander le système de mesure (17) de manière à ce que le système de mesure (17) détecte la première surface extérieure (21u) de la lenticule (21) produite par la première découpe de tissu, et pour commander le système de balayage (100) de manière à ce que le système de balayage (100) dirige le faisceau laser pulsé vers des points cibles de traitement (F) sur une seconde surface extérieure (21o) de la lenticule (21), destinée à être découpée, positionnée par rapport à la première surface extérieure (21u) détectée de la lenticule (21) afin de produire une seconde découpe de tissu pour découper le lenticule (21), positionnée par rapport à la première surface extérieure (21u) détectée de la lenticule (21).

2. Dispositif ophtalmologique (1) selon la revendication 1, **caractérisé en ce que** le circuit (10) est conçu pour positionner la seconde surface extérieure (21o) de la lenticule (21), destinée à être découpée, avec une épaisseur centrale prédéterminée (d) de la lenticule (21), destinée à être découpée, par rapport à la première surface extérieure (21u) détectée.

3. Dispositif ophtalmologique (1) selon l'une des revendications 1 ou 2, **caractérisé en ce que** le circuit (10) est conçu pour positionner la seconde surface extérieure de la lenticule (21), destinée à être découpée, avec un profil d'épaisseur prédéterminé (D) de la lenticule (21), destinée à être découpée, par rapport à la première surface extérieure (21u) détectée.

4. Dispositif ophtalmologique (1) selon l'une des revendications 1 à 3, **caractérisé en ce que** le circuit (10) est conçu pour commander le système de mesure (17) de manière à ce que le système de mesure (17) détecte des déformations de la première surface extérieure (21u), provoquées par des bulles de gaz générées pendant la première découpe de tissu, et positionne la seconde surface extérieure (21o) de la lenticule (21), destinée à être découpée, par rapport à la première surface extérieure (21u) détectée en tenant compte des déformations détectées.

5. Dispositif ophtalmologique (1) selon l'une des revendications 1 à 4, **caractérisé en ce que** le circuit (10) est conçu pour déterminer la première surface extérieure (21u) de la lenticule (21), destinée à être découpée, sous la forme d'une surface inférieure de la lenticule (21), destinée à être découpée, tournée à l'opposé d'une surface cornéenne extérieure (200), et pour déterminer la seconde surface extérieure (21o) de la lenticule (21), destinée à être découpée, sous la forme d'une surface supérieure de la lenticule (21), destinée à être découpée, tournée vers la surface cornéenne extérieure (200).

6. Dispositif ophtalmologique (1) selon l'une des revendications 1 à 5, **caractérisé en ce que** le circuit (10) est conçu pour commander le système de balayage (100) de manière à ce que le système de balayage (100) dirige le faisceau laser pulsé vers des points cibles de traitement (F) selon des trajets de traitement adjacents (3) sur la première surface extérieure (21u) de la lenticule (21), destinée à être découpée, afin de produire des trajets de découpe adjacents (30) de la première découpe de tissu ; et pour commander le système de mesure (17) de manière à ce que le système de mesure (17) détecte les trajets de découpe adjacents (30) et positionne la seconde surface extérieure (21o) de la lenticule (21), destinée à être découpée, par rapport aux trajets de découpe adjacents (30) détectés.

7. Dispositif ophtalmologique (1) selon la revendication 6, **caractérisé en ce que** le circuit (10) est conçu pour commander le système de balayage (100) de manière à ce que le système de balayage (100) dirige le faisceau laser pulsé vers des points cibles de traitement (F) selon des trajets de traitement adjacents (3) sur la première surface extérieure (21u) de la lenticule (21), destinée à être découpée, afin de produire des trajets de découpe adjacents (30) de la première découpe de tissu, séparés par des ponts de tissu restants (31) ; pour commander le système de mesure (17) de manière à ce que le système de mesure (17) détecte les trajets de découpe adjacents (30) ; et pour commander le système de balayage (100) de manière à ce que le système de balayage (100) dirige le faisceau laser pulsé vers des points cibles de traitement (F) dans les ponts de tissu restants (31) sur la première surface extérieure (21u) déterminée sur la base des trajets de découpe adjacents (30) détectés afin de produire la première découpe de tissu.

8. Dispositif ophtalmologique (1) selon l'une des revendications 6 ou 7, **caractérisé en ce que** le circuit (10) est conçu pour commander le système de balayage (100) de manière à ce que le système de balayage (100) dirige le faisceau laser pulsé vers des points cibles de traitement (F) selon des trajets de traitement (3) qui s'étendent sur la surface extérieure (21o, 21u) de la lenticule (21) et présentent l'une des configurations suivantes: rectiligne, le long de cercles, circulaire concentrique, elliptique concentrique, spirale et bras de spirale.

9. Dispositif ophtalmologique (1) selon l'une des revendications 1 à 8, **caractérisé en ce que** le circuit (10) est conçu pour commander un second module de balayage (15) du système de balayage (15) de manière à ce que le second module de balayage (15) dirige le faisceau laser pulsé vers des points cibles de traitement (F) dans une direction d'avance (v) le long d'une ligne de traitement (s) s'étendant respectivement sur les première ou seconde surfaces extérieures (21u, 21o) de la lenticule (21), destinée à être découpée ; **en ce que** le système de balayage comprend un premier module de balayage (12) qui est conçu pour diriger le faisceau laser pulsé le long d'une ligne de balayage (f) s'étendant sous un angle d'orientation (δ) transversalement à la ligne de traitement (s) dans un plan de traitement horizontal (x/y), à une vitesse de balayage sensiblement plus élevée dans la direction d'avance (v) par rapport à la vitesse de balayage du second module de balayage (15) ; et **en ce que** le système de balayage comprend un modulateur z (13) qui est conçu pour faire basculer la ligne de balayage (f) hors du plan de travail (x/y) en fonction d'un point cible de traitement déterminé du second module de balayage (15) sur la ligne de traitement (s) de manière à ce que la ligne de balayage (f) s'étende sensiblement le long de la surface extérieure (21o, 21u) de la lenticule (21).

10. Dispositif ophtalmologique (1) selon l'une des revendications 1 à 9, **caractérisé en ce que** le système de mesure (17) est réalisé sous la forme d'un système de mesure interférométrique.
